# EUROPEAN PATENT APPLICATION

(11) **EP 4 201 385 A1**
(43) Date of publication of application: **28.06.2023**
(21) Application number: 21875644.3
(22) Date of filing: 28.09.2021
(51) Int. Cl.: A61F 13/534, A61F 13/535, A61F 13/536

(54) **ABSORBENT ARTICLE**

(30) Priority: 29.09.2020 JP 2020164146
(71) Applicant: Unicharm Corporation, Ehime 799-0111 (JP)
(72) Inventor: MUKAI, Hirotomo, Kanonji-shi, Kagawa 769-1602 (JP); OHNISHI, Kazuaki, Kanonji-shi, Kagawa 769-1602 (JP)
(74) Representative: Dolleymores
(86) International application number: PCT/JP2021/035705
(87) International publication number: WO 2022/071343

(57) **Abstract**

In an absorbent article (1) having an upper layer core (31) and a lower layer core (32), the lower layer core (32) has a groove (36) which is indented in the thickness direction and the length of which is in a prescribed direction, the lower layer core (32) has a groove region where the groove (36) is provided and a non-groove region where the groove (36) is not provided, at least a part of the groove region is provided in a region where the upper layer core (31) and lower layer core (32) overlap when viewed from the thickness direction, and the average density of a highly absorbent polymer (34) on a skin-side section of the groove region is lower than the average density of a highly absorbent polymer (34) on a skin-side section of the non-groove region.

## Description

### [Technical Field]

The present invention relates to an absorbent article.

### [Background Art]

Patent Document 1 discloses an absorbent article that includes an absorbent body having a two-layer structure including an upper-layer absorbent body and a lower-layer absorbent body. A slit that penetrates at least the upper-layer absorbent body in the thickness direction is provided at the widthwise center of the upper-layer absorbent body. Also, the weight ratio of super absorbent polymer to pulp fiber is higher in the upper-layer absorbent body than in the lower-layer absorbent body. According to this configuration, excreted fluid is supplied to the upper-layer absorbent body as well to a certain extent, but is preferentially supplied to the lower-layer absorbent body through the slit. After the lower-layer absorbent body becomes saturated, the liquid absorbed by the lower-layer absorbent body is sucked up by the upper-layer absorbent body and retained by the super absorbent polymer of the upper-layer absorbent body. For this reason, this configuration is deemed to have excellent rewetting (backflow) prevention.

### [Citation List]

### [Patent Literature]

[Patent Document 1] Japanese Patent Application Publication No. 2017-63923

### [Summary of Invention]

### [Technical Problem]

In an absorbent body having a two-layer structure, it is possible to increase the absorption capacity. But, the excreted fluid is more likely to accumulate in spaces at the boundary between an upper absorbent body (upper-layer core) and a lower absorbent body (lower-layer core) in the thickness direction. In this case, the amount of excreted fluid that is absorbed by the lower absorbent body decreases, making impossible to effectively utilize the absorption performance of the lower absorbent body. In addition, if a relatively large slit is provided in the upper-layer absorbent body (upper-layer core) as in Patent Document 1, the excreted fluid is more likely to accumulate. In Patent Document 1, the weight ratio of super absorbent polymer in the lower absorbent body decreases, and thereby the excreted fluid is absorbed and dispersed over a wider range. But, there is a limit to absorption and dispersion, by the lower absorbent body, of the excreted fluid which has been locally supplied through the slit. The ability of excreted fluid to disperse in the lower absorbent body is insufficient.

The present invention was achieved in light of conventional problems such as that described above, and an aspect of the present invention is to provide an absorbent article that includes an upper-layer core and a lower-layer core overlaid on each other, that suppresses the accumulation of excreted fluid at the boundary between the upper-layer core and the lower-layer core, and that effectively utilize the absorption performance of the lower absorbent body by increasing the ability of excreted fluid to disperse in the lower absorbent body.

### [Solution to Problem]

A main aspect of the present invention for achieving the above-described aspect is an absorbent article having a longitudinal direction, a width direction, and a thickness direction, the absorbent article including: an absorbent core, the absorbent core including an upper-layer core and a lower-layer core, the lower-layer core being arranged on a non-skin side in the thickness direction with respect to the upper-layer core, the lower-layer core including a super absorbent polymer, the lower-layer core having a groove in which a portion of the lower-layer core is recessed in the thickness direction, the groove being elongated in a predetermined direction, when viewed in the thickness direction, the lower-layer core having a groove region in which the groove is provided and a non-groove region in which the groove is not provided, when viewed in the thickness direction, at least a portion of the groove region being provided in a region where the upper-layer core and the lower-layer core are overlapped with each other, when the lower-layer core is divided into two portions in the thickness direction, an average density of the super absorbent polymer in a skin-side portion of the groove region being lower than an average density of the super absorbent polymer in a skin-side portion of the non-groove region, the skin-side portion being a portion located on a skin side, the non-skin-side portion being a portion located on a non-skin side.

Features of the present invention other than the above will become clear by reading the description of the present specification with reference to the accompanying drawings.

### [Advantageous Effects of Invention]

According to the present invention, it is possible to provide an absorbent article that includes an upper-layer core and a lower-layer core overlaid on each other, that suppresses the accumulation of excreted fluid at the boundary between the upper-layer core and the lower-layer core, and that effectively utilize the absorption performance of the lower absorbent body by increasing the ability of excreted fluid to disperse in the lower absorbent body.

### [Brief Description of the Drawings]

FIG. 1 is a schematic perspective view of a pull-on diaper 1 (hereinafter, also referred to as a "diaper").
FIG. 2 is a schematic plan view of the diaper 1 in an unfolded and stretched state as viewed from the skin side.
FIG. 3 is a schematic cross-sectional view taken along a line a-a in FIG. 2.
FIG. 4 is a plan view of an upper-layer core 31 as viewed from the skin side in the thickness direction.
FIG. 5 is a plan view of a lower-layer core 32 as viewed from the skin side in the thickness direction.
FIG. 6 is a plan view of the upper-layer core 31 and the lower-layer core 32 in an overlaid state as viewed from the skin side in the thickness direction.
FIG. 7 is a schematic cross-sectional view of a portion of the upper-layer core 31 and the lower-layer core 32.
FIG. 8A is a schematic cross-sectional view taken along a line I-I in FIG. 6, and FIG. 8B is a schematic cross-sectional view taken along a line II-II in FIG. 6.
FIG. 9A is a schematic perspective view of a core-wrapping sheet 39, and FIG. 9B is an illustrative view of a variation of the arrangement of the core-wrapping sheet 39.
FIGS. 10A and 10B are illustrative views of variations of grooves 35 and grooves 36.
FIGS. 11A and 11B are illustrative views of variations of grooves 35 and grooves 36.
FIG. 12 is a schematic cross-sectional view of an absorbent core 30 according to a second embodiment.
FIG. 13 is a schematic cross-sectional view of the absorbent core 30 according to a third embodiment.
FIGS. 14A and 14B are schematic cross-sectional views of the absorbent core 30 according to a fourth embodiment.
FIG. 15 is a schematic plan view of the diaper 1 according to a fifth embodiment in an unfolded and stretched state, as viewed from the skin side.
FIG. 16 is a schematic cross-sectional view taken along A-A in FIG. 15.
FIG. 17 is a plan view of the upper-layer core 31 and the lower-layer core 32 in an overlaid state as viewed from the non-skin side in the thickness direction.
FIG. 18A is a plan view of the upper-layer core 31 as viewed from the skin side in the thickness direction. FIG. 18B is a plan view of the lower-layer core 32 as viewed from the non-skin side in the thickness direction.
FIG. 19 is a plan view of the absorbent core 30 in a variation of the fifth embodiment, as viewed from the non-skin side in the thickness direction.

### [Description of Embodiments]

At least the following matters will become clear with the description of this specification and the attached drawings. An absorbent article having a longitudinal direction, a width direction, and a thickness direction, the absorbent article including: an absorbent core, the absorbent core including an upper-layer core and a lower-layer core, the lower-layer core being arranged on a non-skin side in the thickness direction with respect to the upper-layer core, the lower-layer core including a super absorbent polymer, the lower-layer core having a groove in which a portion of the lower-layer core is recessed in the thickness direction, the groove being elongated in a predetermined direction, when viewed in the thickness direction, the lower-layer core having a groove region in which the groove is provided and a non-groove region in which the groove is not provided, when viewed in the thickness direction, at least a portion of the groove region being provided in a region where the upper-layer core and the lower-layer core are overlapped with each other, when the lower-layer core is divided into two portions in the thickness direction, an average density of the super absorbent polymer in a skin-side portion of the groove region being lower than an average density of the super absorbent polymer in a skin-side portion of the non-groove region, the skin-side portion being a portion located on a skin side, the non-skin-side portion being a portion located on a non-skin side.

According to this absorbent article, the excreted fluid that has passed through the upper-layer core can be drawn into the groove of the skin-side portion of the lower-layer core, in which the average density of the super absorbent polymer is low, or can drop into the space of the groove. Accordingly, the excreted fluid can be dispersed in the predetermined direction along the groove region. This makes it possible to suppress the accumulation of the excreted fluid at the boundary between the upper-layer core 31 and the lower-layer core 32, making it possible to increase the ability to disperse excreted fluid in the lower-layer core. Accordingly, the absorption performance of the lower-layer core 32 can be effectively utilized. Also, because the excreted fluid absorbed in the skin-side portion of the non-groove region of the lower-layer core is held by the super absorbent polymer, the rewetting of the excreted fluid to the upper-layer core can be suppressed.

In such an absorbent article, the absorbent core has a widthwise central portion and a pair of widthwise side portions, the widthwise central portion and the pair of widthwise side portions being defined by dividing a maximum width of the absorbent core into three portions in the width direction, the absorbent core has a front portion located on a front side in the longitudinal direction with respect to a product center line and a back portion located on a back side in the longitudinal direction with respect to the product center line, the product center line being a line that divides the absorbent article in the unfolded and stretched state into two portions in the longitudinal direction, the back portion has a back first region that is a central region obtained by dividing a maximum length of the back portion into three portions in the longitudinal direction, the front portion has a front first region that is a central region obtained by dividing a maximum length of the front portion into three portions in the longitudinal direction, concerning a value obtained by dividing an area of the groove region of the lower-layer core located in the widthwise central portion in the back first region by an area of the widthwise central portion in the back first region, concerning a value obtained by dividing an area of the groove region of the lower-layer core located in the widthwise central portion in the front first region by an area of the widthwise central portion in the front first region, the former value is smaller than the latter value.

According to this absorbent article, the area ratio of the groove region of the lower-layer core is small in the widthwise central portion of the back first region, where the body weight of the wearer is likely to be applied, and therefore the amount of excreted fluid from the groove region that causes rewetting can be reduced. On the other hand, in the front first region, which is a portion that comes into contact with the excretion source or a portion close thereto, the area ratio of the groove regions of the lower-layer core is large, excreted fluid that has passed through the upper-layer core can be dispersed while being quickly drawn into the lower-layer core by the groove regions. This makes it possible to suppress the case where excreted fluid is accumulated at the boundary between the upper-layer core and the lower-layer core, and the excreted fluid fails to be completely absorbed by the upper-layer core and flows beyond the skin-side surface of the absorbent article and leaks.

In such an absorbent article, a portion of the lower-layer core located in the widthwise central portion in the back first region has a region provided with only one groove that is elongated in the longitudinal direction, the longitudinal direction being the predetermined direction.

According to this absorbent article, the widthwise central portion of the back first region, where the body weight of the wearer is likely to be applied, includes a region where a plurality of groove regions are not arranged side by side in the width direction, and therefore rewetting can be suppressed. Also, excreted fluid flowing from the portion in contact with the excretion source to the back first region passes through the upper-layer core 31 and then is dispersed while being absorbed into the lower-layer core by the groove regions of the lower-layer core. This makes it possible to suppress the leakage of excreted fluid.

In such an absorbent article, an average basis weight of the absorbent core is higher in the widthwise central portion in the back first region than in the pair of widthwise side portions in the back first region, and portions of the lower-layer core respectively located in the pair of widthwise side portions in the back first region has a groove that is elongated in the longitudinal direction, the longitudinal direction being the predetermined direction.

According to this absorbent article, the absorption and retention capacity for excreted fluid is increased in the widthwise central portion in the back first region, where the body weight of the wearer is likely to be applied, and therefore rewetting can be suppressed. Also, excreted fluid that has flowed from the portion in contact with the excretion source to the back first region can be dispersed in the longitudinal direction while being quickly drawn into the lower-layer core by the groove regions of the lower-layer core located in the widthwise side portions. This makes it possible to suppress the leakage (lateral leakage) of excreted fluid.

In such an absorbent article, the back portion has a back second region on the front side in the longitudinal direction with respect to the back first region, the front portion has a front second region on the back side in the longitudinal direction with respect to the front first region, and the lower-layer core has a region in which the non-groove region is continuous from one end to another end in the width direction, between the groove region of the lower-layer core located in the back second region and the groove region of the lower-layer core located in the front second region, with respect to the longitudinal direction.

According to this absorbent article, a large amount of excreted fluid can be dispersed while being quickly absorbed into the lower-layer core from the upper-layer core by the groove region of the lower-layer core located in the front second region, which is a portion in contact with the excretion source or a portion close thereto. Absorbed excreted fluid can be retained in the non-groove region, and rewetting can be suppressed. Also, excreted fluid that has not been completely absorbed by the front second region and has flowed backward can be dispersed while being absorbed into the lower-layer core by the groove region of the lower-layer core located in the back second region. This makes it possible to suppress the leakage of excreted fluid.

In such an absorbent article, the back portion has a back second region on the front side in the longitudinal direction with respect to the back first region, and concerning a value obtained by dividing an area of the groove region of the lower-layer core located in the widthwise central portion in the back second region by an area of the widthwise central portion in the back second region, concerning the value obtained by dividing the area of the groove region of the lower-layer core located in the widthwise central portion in the back first region by the area of the widthwise central portion in the back first region, the former value is greater than the latter value.

According to this absorbent article, the area ratio of the groove region of the lower-layer core is small in the widthwise central portion of the back first region, where the body weight of the wearer is likely to be applied, and therefore rewetting can be suppressed. On the other hand, the area ratio of the groove region of the lower-layer core is large in the back second region, which is a portion close to the portion in contact with the excretion source, and therefore excreted fluid that has passed through the upper-layer core can be dispersed while being quickly drawn into the lower-layer core by the groove region of the lower-layer core. This makes it possible to suppress the leakage of excreted fluid.

In such an absorbent article, the front portion has a front second region on the back side in the longitudinal direction with respect to the front first region, and a portion of the lower-layer core located in the widthwise central portion in the front first region has more number of grooves that are elongated in the longitudinal direction than a portion of the lower-layer core located in the widthwise central portion in the front second region, the longitudinal direction being the predetermined direction.

According to this absorbent article, compared with the front second region that comes into contact with the wearer's crotch region, the front first region is less likely to be subjected to the body weight of the wearer, and therefore rewetting is not likely to occur even if the number of grooves is higher. Also, in the case where the wearer is a male, the front first region is a portion that comes into contact with the excretion source or a portion close thereto, and therefore excreted fluid that has excreted from the excretion source and has passed through the upper-layer core can be dispersed while being quickly drawn into the lower-layer core by the many groove regions of the lower-layer core. This makes it possible to suppress the leakage of excreted fluid.

In such an absorbent article, an average basis weight of the lower-layer core is higher than an average basis weight of the upper-layer core.

According to this absorbent article, the absorption and retention capacity of the upper-layer core is relatively small, and excreted fluid that has passed through the upper-layer core can be dispersed while being quickly drawn into the lower-layer core by the groove region of the lower-layer core. This makes it possible to more effectively suppress the leakage of excreted fluid.

In such an absorbent article, the absorbent article further comprises a pair of leak-proof wall portions in two widthwise side portions of the absorbent core, the pair of leak-proof wall portions being capable of rising to the skin side, and the lower-layer core has the groove region that is at least partially overlapped with the leak-proof wall portion when the absorbent article in an unfolded and stretched state is viewed in the thickness direction.

According to this absorbent article, excreted fluid that is blocked by the leak-proof wall portion and absorbed by the upper-layer core can be dispersed while being quickly drawn into the lower-layer core by the groove region of the lower-layer core. Also, in the portion including the leak-proof wall portion, the leak-proof wall portion can function as a cushioning material, and is not likely to be subjected to body weight, and therefore rewetting of the groove region can be suppressed.

In such an absorbent article, the upper-layer core includes a super absorbent polymer, and an average density of the super absorbent polymer in the upper-layer core is higher than an average density of the super absorbent polymer in the lower-layer core.

According to this absorbent article, even if rewetting of the upper-layer core occurs due to excreted fluid absorbed by the lower-layer core (in particular, rewetting of excreted fluid to the upper-layer core from the groove region of the lower-layer core is likely to occur), the excreted fluid is retained by the super absorbent polymer in the upper-layer core. Therefore rewetting at the skin-side surface of the absorbent article can be suppressed.

In such an absorbent article, an average density of the super absorbent polymer in a non-skin-side portion of the non-groove region is lower than the average density of the super absorbent polymer in the skin-side portion of the non-groove region.

According to this absorbent article, excreted fluid that has passed through the groove region of the lower-layer core and moved to the non-skin-side portion is likely to be absorbed by the non-skin-side portion of the non-groove region where the density of the super absorbent polymer is low. Accordingly, the ability of excreted fluid to disperse in the non-skin-side portion of the lower-layer core is improved. Also, excreted fluid that has moved to the non-skin-side portion of the non-groove region is sucked up by the skin-side portion of the non-groove region and is held by the super absorbent polymer, thus making it possible to suppress the rewetting of excreted fluid to the upper-layer core.

In such an absorbent article, the groove is a groove that is recessed from a non-skin-side surface of the lower-layer core toward the skin side in the thickness direction.

According to this absorbent article, the ability of liquid to permeate to the non-skin-side portion in the groove region of the lower-layer core (to the space of the groove) is improved. For this reason, after excreted fluid has been reliably drawn to the non-skin-side portion of the lower-layer core, the excreted fluid can be dispersed along the groove, and the absorption performance of the lower-layer core can be effectively utilized.

In such an absorbent article,
the upper-layer core includes a super absorbent polymer, and when the upper-layer core is divided into two portions in the thickness direction,
in at least a portion of the lower-layer core, an average density of the super absorbent polymer in the skin-side portion in a given region of the lower-layer core is higher than an average density of the super absorbent polymer in a non-skin-side portion in a region of the upper-layer core that faces the given region, the skin-side portion being a portion located on a skin side, the non-skin-side portion being a portion located on a non-skin side.

According to this absorbent article, the speed of movement of fluid from the upper-layer core to the lower-layer core can be suppressed appropriately. For this reason, the ability of excreted fluid to disperse in the upper-layer core is improved, and the excreted fluid is absorbed over a wide range in the lower-layer core that receives the excreted fluid.

In such an absorbent article,
the groove includes
a main groove that is elongated in the predetermined direction that is the longitudinal direction, and
a sub groove that extends from the main groove in the width direction,
a plurality of the main grooves are arranged side-by-side spacing in the width direction, and
a plurality of the sub grooves extend from each of the main grooves in the width direction with located spacing in the longitudinal direction.

According to this absorbent article, excreted fluid can be dispersed in the longitudinal direction in the lower-layer core and also dispersed in the width direction along the sub groove. Also, the flexibility of the lower-layer core increases, and the lower-layer core can easily deform along the wearer's body.

In such an absorbent article,
the upper-layer core includes a super absorbent polymer, and
the upper-layer core has a second groove in which a portion of the upper-layer core is recessed in the thickness direction,
the second groove is elongated in the longitudinal direction,
when viewed in the thickness direction, the upper-layer core has a second groove region in which the second groove is provided and
a second non-groove region in which the second groove is not provided,
when viewed in the thickness direction, at least a portion of the second groove region is provided in a region where the upper-layer core and the lower-layer core are overlapped with each other, and
when the upper-layer core is divided into two portions in the thickness direction,
an average density of the super absorbent polymer in a skin-side portion of the second groove region is lower than an average density of the super absorbent polymer in a skin-side portion of the second non-groove region,
the skin-side portion being a portion located on a skin side, the non-skin-side portion being a portion located on a non-skin side.

According to this absorbent article, fluid excreted on the skin-side surface of the upper-layer core can be drawn into the skin-side portion of the second groove region, in which the average density of the super absorbent polymer is low, or can drop into the space of the second groove region. This increases the ability to disperse excreted fluid in the upper-layer core. Also, the ability to disperse excreted fluid in the lower-layer core increases because the lower-layer core receives and absorbs the excreted fluid that has been dispersed in the upper-layer core. Also, because the excreted fluid absorbed in the skin-side portion of the second non-groove region is held by the super absorbent polymer, the rewetting of the excreted fluid to the skin-side surface of the upper-layer core can be suppressed.

In such an absorbent article, an average density of the super absorbent polymer in the non-skin-side portion of the second non-groove region is lower than an average density of the super absorbent polymer in the skin-side portion of the second non-groove region.

According to this absorbent article, excreted fluid that has passed through the second groove region of the upper-layer core and moved to the non-skin-side portion is likely to be absorbed by the non-skin-side portion of the second non-groove region where the density of the super absorbent polymer is low. Accordingly, the ability of excreted fluid to disperse in the non-skin-side portion of the upper-layer core is improved. Also, excreted fluid that has moved to the non-skin-side portion of the second non-groove region is sucked up by the skin-side portion of the second non-groove region and held by the super absorbent polymer, and thus the rewetting of excreted fluid to the skin-side surface of the upper-layer core can be suppressed.

In such an absorbent article, the second groove is a groove that is recessed from a non-skin-side surface of the lower-layer core toward the skin side in the thickness direction.

According to this absorbent article, fluid excreted in the upper-layer core can immediately drop into the space of the second groove of the upper-layer core and be dispersed. Accordingly, it is possible to suppress the case where excreted fluid overflows on the skin-side surface of the upper-layer core.

In such an absorbent article, the groove region of the lower-layer core has a portion that is overlapped with the second groove region of the upper-layer core when viewed in the thickness direction.

According to this absorbent article, a large amount of excreted fluid that has been absorbed in the second groove region of the upper-layer core can be drawn into the groove region of the lower-layer core. Accordingly, it is possible to suppress the case where excreted fluid overflows on the skin-side surface of the upper-layer core.

In such an absorbent article,
the lower-layer core has a slit in a widthwise central portion of a region of the lower-layer core that abuts a crotch portion of a wearer,
the slit penetrating the lower-layer core in the thickness direction,
the slit is elongated in the longitudinal direction, and when viewed in the thickness direction,
the slit of the lower-layer core is overlapped with the upper-layer core, and
in at least a partial region of the slit of the lower-layer core, the slit is not overlapped with the second groove region of the upper-layer core.

According to this absorbent article, the slit makes the widthwise central portion of the absorbent core easier to deform so as to protrude toward the skin side, improving the fit of the absorbent core to the wearer's crotch region. Also, a portion of the absorbent core provided with the slit is overlapped with the second non-groove region of the upper-layer core, thus making it possible to prevent the stiffness of the absorbent core from being excessively lowered, and to suppress the loss of the shape of the absorbent core.

In such an absorbent article,
an elastic member is arranged in a region that abuts the crotch portion of the wearer,
the elastic member stretching and contracting in the longitudinal direction,
an entirety of the slit of the lower-layer core is not overlapped with the second groove region of the upper-layer core when viewed in the thickness direction, and
the slit has a portion overlapped with the elastic member when viewed in the thickness direction.

According to this absorbent article, the slit and the groove region of the upper-layer core are not overlapped with each other in the thickness direction, thus making it less likely for excreted fluid to move from the upper-layer core to the slit, and making it possible to suppress the accumulation of excreted fluid in the slit. Also, deformation of the absorbent core so as to protrude toward the skin side due to the slit of the lower-layer core is promoted by the elastic member. For this reason, the fit of the absorbent core to the wearer's crotch region is improved, and the slit portion of the lower-layer core bends so as to suppress the accumulation of excreted fluid.

In such an absorbent article,
the lower-layer core has a pair of side slits in widthwise side portions of a region of the lower-layer core that abuts a crotch portion of a wearer,
the pair of side slits penetrating the lower-layer core in the thickness direction,
the groove region is provided at a position
that are outward in the width direction with respect to the pair of side slits,
that are at least partially overlapped with the pair of side slits in the longitudinal direction.

According to this absorbent article, due to the side slits of the lower-layer core, the widthwise side portions of the absorbent core rise to the skin side, making it easier to fit to the wearer's upper leg joints. The widthwise side portions of the absorbent core that have risen have higher flexibility due to the groove region, making it possible to easily deform along the wearer's upper leg joints, and improving the fit.

In such an absorbent article,
the lower-layer core has a slit in a widthwise central portion of a region of the lower-layer core that abuts a crotch portion of a wearer,
the slit penetrating the lower-layer core in the thickness direction,
the slit is elongated in the longitudinal direction,
the groove region is elongated in the predetermined direction that is the longitudinal direction,
at a position backward in the longitudinal direction with respect to the slit,
the lower-layer core has a region in which the non-groove region is continuous from one end to another end in the width direction, and
a back end of the upper-layer core in the longitudinal direction is located in the region in which the non-groove region is continuous.

According to this absorbent article, the lower-layer core can easily bend in the longitudinal direction along the back end of the upper-layer core. Accordingly, the back end portion of the lower-layer core can easily conform to the wearer's buttocks region, and the fit of the absorbent core is improved.

In such an absorbent article,
the lower-layer core extends outward in the longitudinal direction from a longitudinal end of the upper-layer core, and the groove region is provided in a portion of the lower-layer core that extends outward in the longitudinal direction from the upper-layer core.

According to this absorbent article, excreted fluid that the lower-layer core has received from the upper-layer core 31 can be dispersed up to the longitudinal end portions of the lower-layer core, which are not overlapped with the upper-layer core in the thickness direction. The end portions of the lower-layer core have higher flexibility due to the groove region, making it possible to easily fit the wearer's lower stomach region and buttocks region.

Hereinafter, a pull-on diaper for adults will be described as an example of an absorbent article according to the present embodiment. However, the absorbent article is not limited to this, and examples of other absorbent articles include tape-type diapers, diapers for children, light incontinence pads, sanitary napkins, and the like.

### Basic Configuration of Pull-on Diaper 1

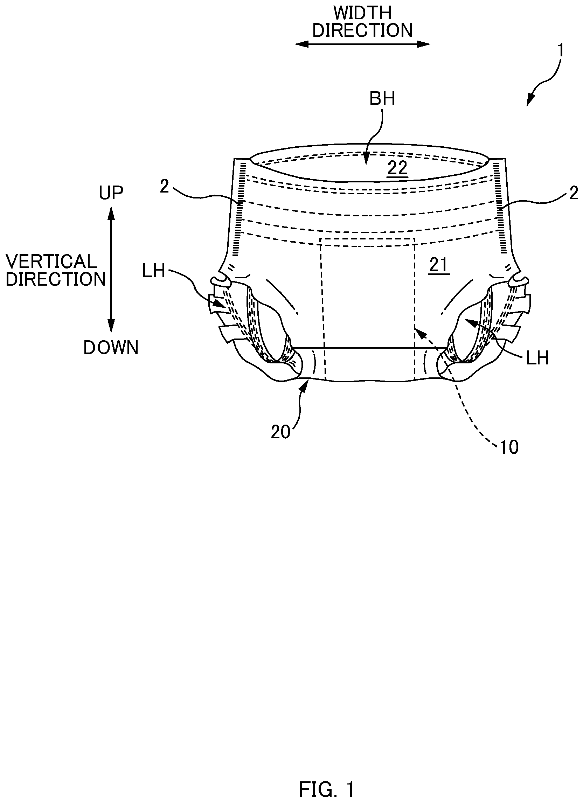

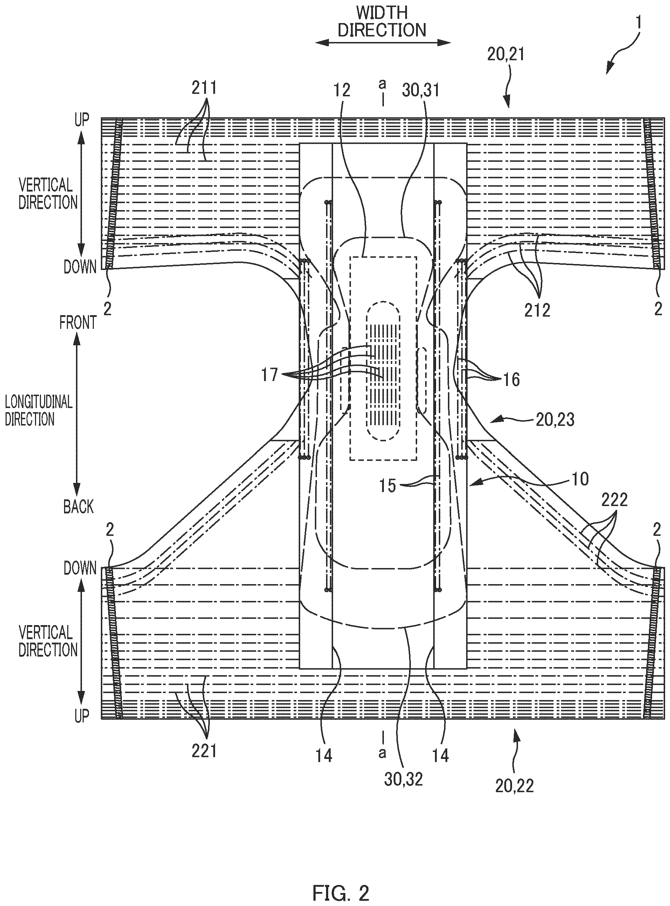

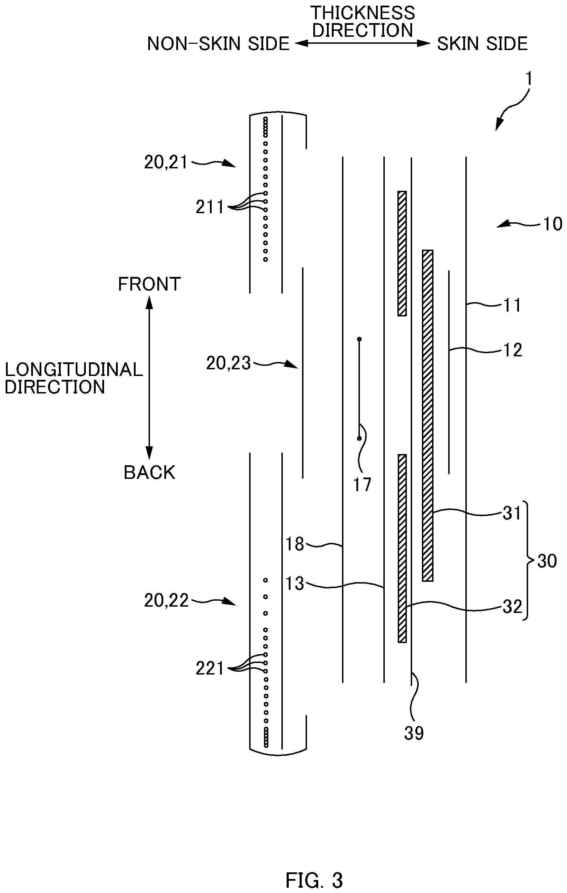

FIG. 1 is a schematic perspective view of a pull-on disposable diaper 1 (hereinafter, also referred to as a "diaper"). FIG. 2 is a schematic plan view of the diaper 1 in an unfolded and stretched state as viewed from the skin side. FIG. 3 is a schematic cross-sectional view taken along a line a-a in FIG. 2.

The unfolded state of the diaper 1 is a state in which joining portions 2 of a front waist portion 21 and a back waist portion 22, which will be described later, have been pulled apart, and the diaper 1 has been unfolded. The stretched state of the diaper 1 is a state in which the diaper 1 has been stretched so as to eliminate wrinkles. Specifically, the diaper 1 is stretched until there is no influence from elastic members of the diaper 1, and the dimensions of the constituent members of the diaper 1 match or are close to the dimensions of the members on their own.

The diaper 1 in the underpants-shaped state has a vertical direction, a width direction, and a thickness direction, and includes an absorbent main body 10 and an exterior member 20. In the vertical direction, the side where a waist opening BH of the diaper 1 is located is the upper side, and the opposite side is the lower side. The longitudinal direction of the diaper 1 in the unfolded state corresponds to the vertical direction of the diaper 1 in the underpants-shaped state, and corresponds to the longitudinal direction of the absorbent main body 10. The side that comes into contact with the wearer's stomach region in the longitudinal direction is the front side, and the side that comes into contact with the wearer's buttocks region is the back side. Also, in the thickness direction, the side that comes into contact with the wearer's skin is the skin side, and the opposite side is the non-skin side.

The exterior member 20 is arranged on the non-skin side of the absorbent main body 10, and includes a front waist portion 21, a back waist portion 22, and a crotch portion 23. The diaper 1 in the unfolded state is folded at approximately the center in the longitudinal direction, and the two widthwise end portions of the front waist portion 21 and the back waist portion 22 are joined by welding or the like to form a pair of joining portions 2, thus obtaining the diaper 1 in the underpants-shaped state.

A plurality of waist elastic members 211 and 221 (e.g., elastic strings) that extend along the width direction are arranged on the front waist portion 21 and the back waist portion 22 spacing in the vertical direction (longitudinal direction). Also, a plurality of leg elastic members 212 and 222 (e.g., elastic strings) are arranged extending along leg openings LH in the front waist portion 21 and the back waist portion 22. The waist elastic members 211 and 221 and the leg elastic members 212 and 222 are fixed in a stretched state between two layers of sheets (e.g., nonwoven fabric sheets).

The crotch portion 23 is constituted by a sheet (e.g., a nonwoven fabric sheet) that connects the front waist portion 21 and the back waist portion 22. However, the exterior member 20 does not need to include the crotch portion 23.

The absorbent main body 10 includes: an absorbent core 30 that absorbs and holds excreted fluid; a liquid-permeable top-face sheet 11 (e.g., a nonwoven fabric sheet or a porous resin film) arranged on the skin side of the absorbent core 30; a liquid-permeable intermediate sheet 12 arranged between the top-face sheet 11 and the absorbent core 30; a liquid-impermeable back sheet 13 (e.g., a resin film) arranged on the non-skin side of the absorbent core 30; a pair of side sheets 14 (e.g., hydrophobic nonwoven fabric sheets) arranged on widthwise side portions of the absorbent main body 10; leak-proof-wall elastic members 15; leg elastic members 16; crotch elastic members 17; and a cover sheet 18 that covers the crotch elastic members 17 from the non-skin side.

Although not shown in detail, the side sheets 14 are folded over from widthwise side portions of the non-skin-side surface of the back sheet 13 toward widthwise side portions of the skin-side surface of the top-face sheet 11. The leak-proof-wall elastic members 15 and the leg elastic members 16 are fixed to the side sheet 14 in a state of being stretched in the longitudinal direction. The leak-proof-wall elastic members 15 are arranged at the leading end portions of the side sheet 14 that were folded inward in the width direction on the skin-side surface of the top-face sheet 11. The leg elastic members 16 are arranged on widthwise side portions of the absorbent main body 10. The crotch elastic members 17 are arranged in a longitudinal and widthwise central portion of the absorbent main body 10, and are fixed between the back sheet 13 and the cover sheet 18 in a state of being stretched in the longitudinal direction.

Although a basic configuration of the pull-on diaper 1 has been described above, the configuration of the pull-on diaper 1 is not limited to the above description.

### Absorbent Main body 10 (Absorbent core 30)

### First Embodiment

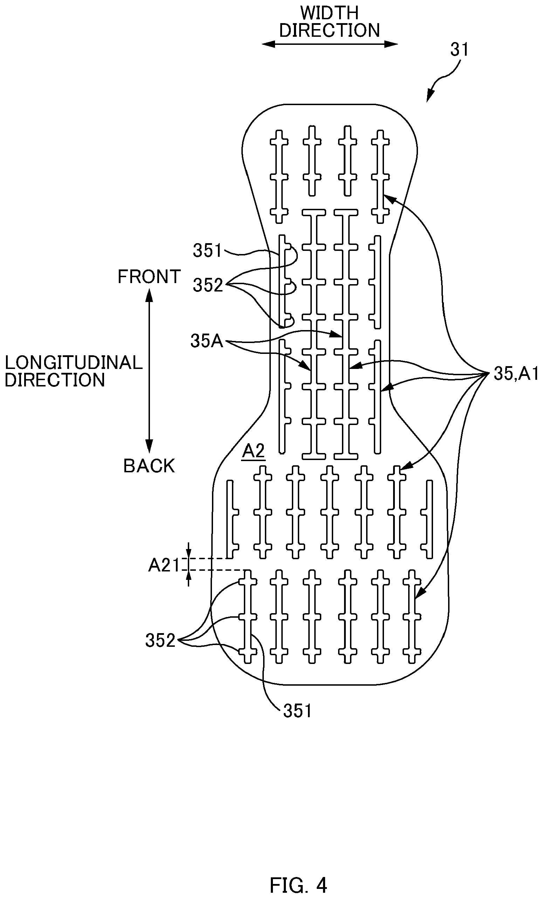

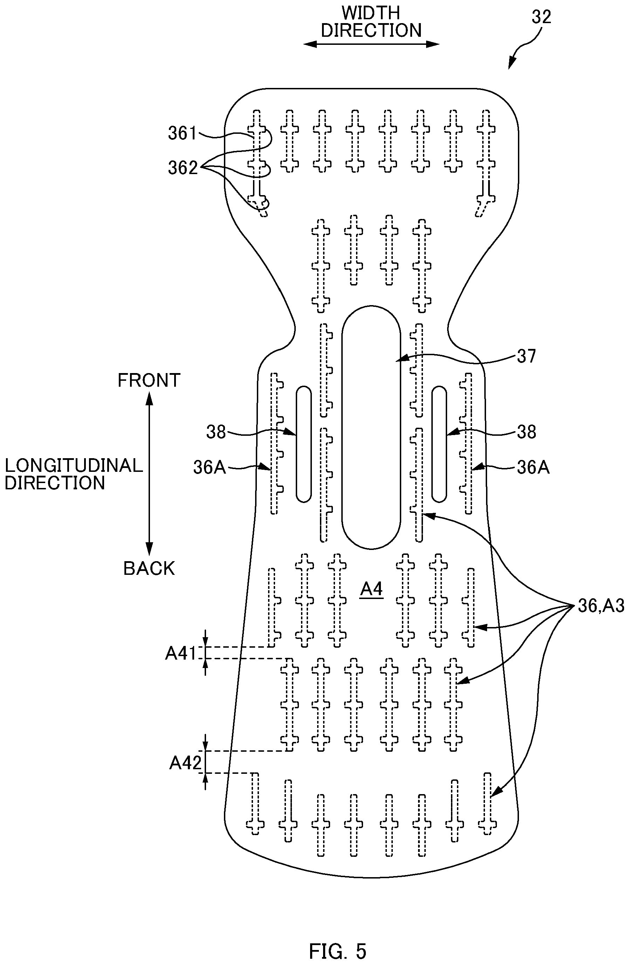

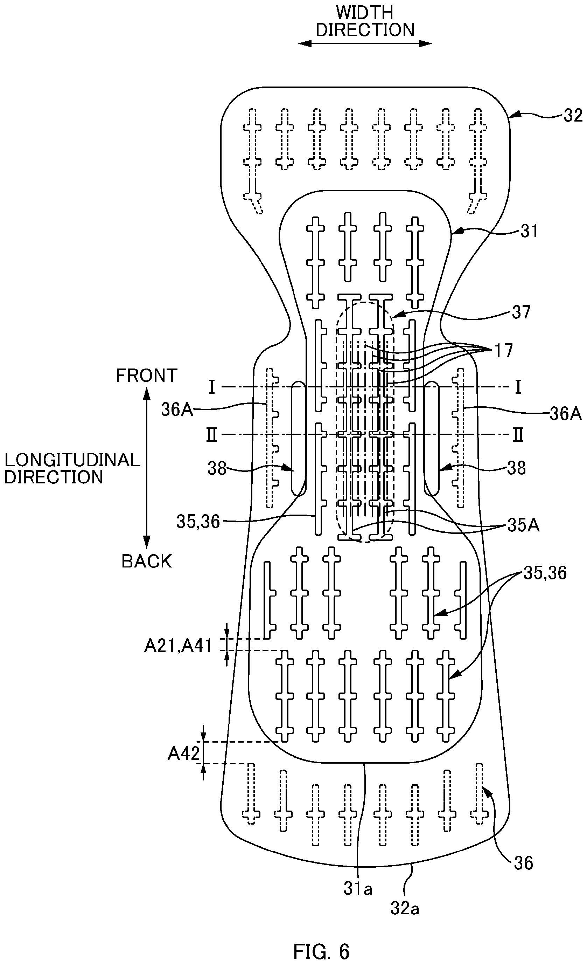

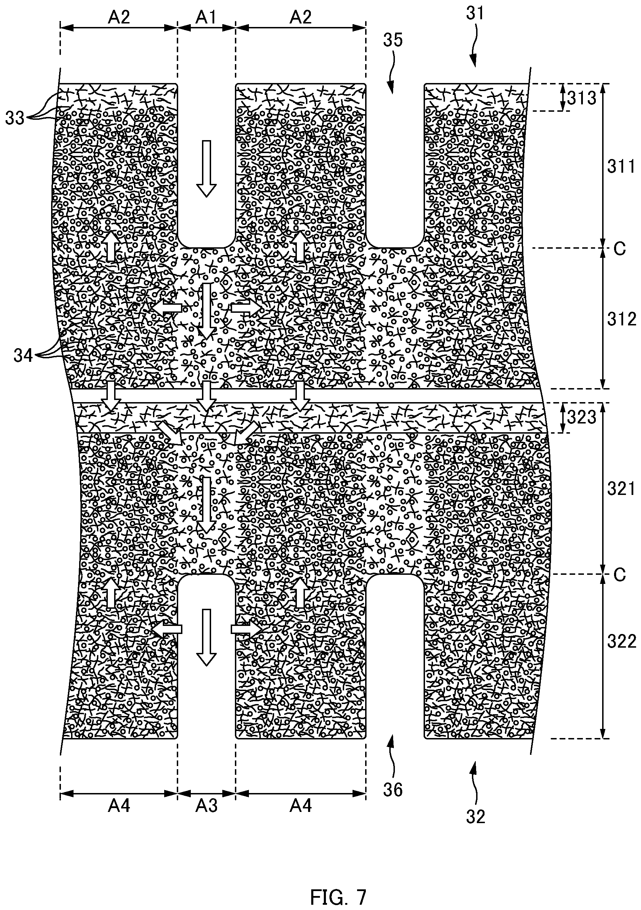

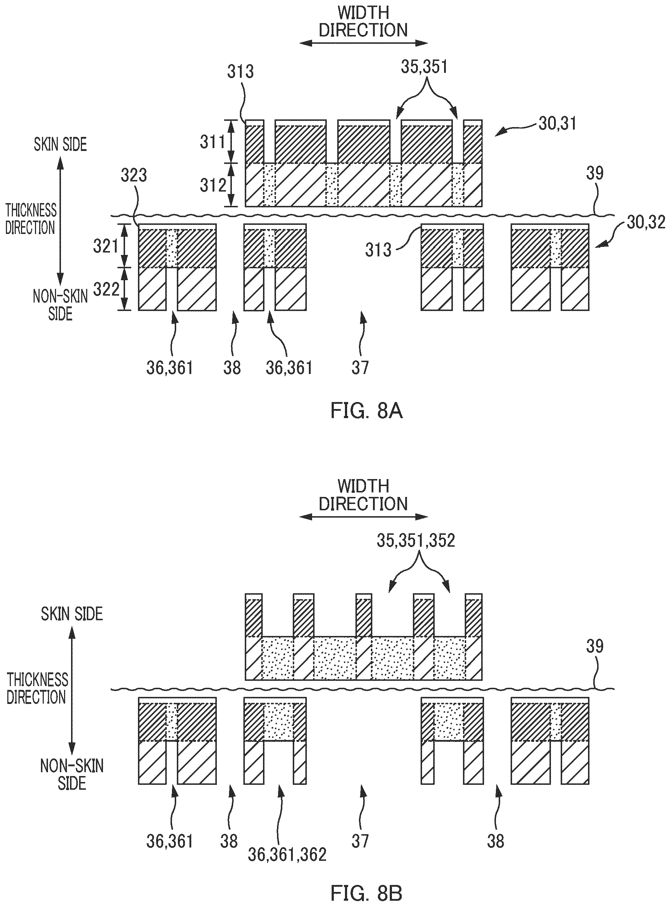

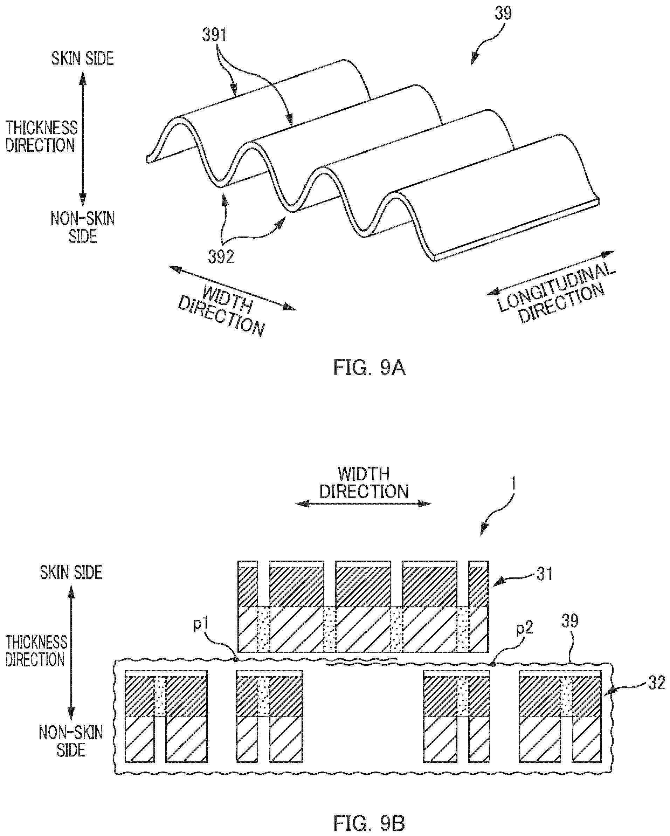

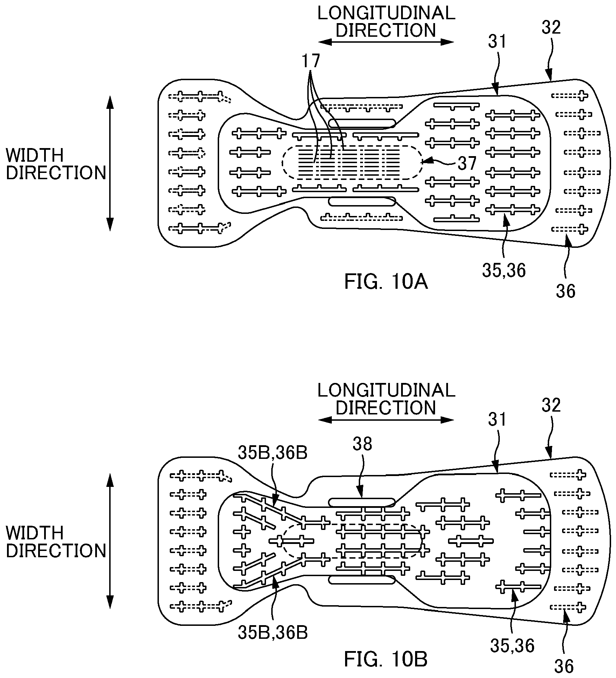

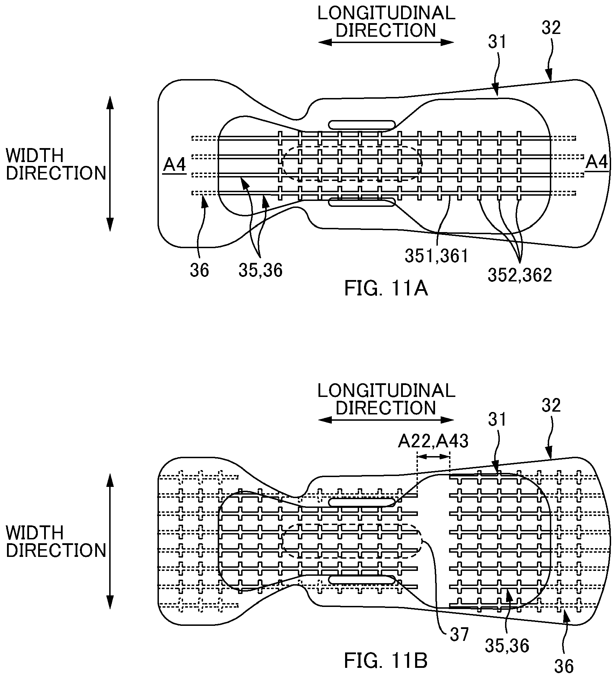

FIG. 4 is a plan view of an upper-layer core 31 as viewed from the skin side in the thickness direction. FIG. 5 is a plan view of a lower-layer core 32 as viewed from the skin side in the thickness direction. FIG. 6 is a plan view of the upper-layer core 31 and the lower-layer core 32 in an overlaid state as viewed from the skin side in the thickness direction. FIG. 7 is a schematic cross-sectional view of a portion of the upper-layer core 31 and the lower-layer core 32. FIG. 8A is a schematic cross-sectional view taken along a line I-I in FIG. 6, and FIG. 8B is a schematic cross-sectional view taken along a line II-II in FIG. 6. FIG. 9A is a schematic perspective view of a core-wrapping sheet 39, and FIG. 9B is an illustrative view of a variation of the arrangement of the core-wrapping sheet 39. FIGS. 10A, 10B, 11A, and 11B are illustrative views of variations of grooves 35 and grooves 36.

The absorbent core 30 has a two-layer structure and includes an upper-layer core 31 and a lower-layer core 32 that is arranged on the non-skin side in the thickness direction with respect to the upper-layer core 31. The upper-layer core 31 and the lower-layer core 32 include liquid absorbent fibers 33 (pulp fiber, cellulosic absorbent fiber, etc.) and a super absorbent polymer 34 (SAP).

The planar shape of the upper-layer core 31 and the lower-layer core 32 is an hourglass shape in which the central portion in the longitudinal direction is constricted inward in the width direction. As shown in FIG. 6, the lower-layer core 32 has a larger size than the upper-layer core 31, and the upper-layer core 31 is arranged so as to fit within the outer peripheral edge of the lower-layer core 32.

The upper-layer core 31 has grooves 35 (second grooves) in which portions of the upper-layer core 31 are recessed in the thickness direction. When viewed in the thickness direction, the upper-layer core 31 has groove regions A1 (second groove regions) in which the grooves 35 are provided, and non-groove regions A2 (second non-groove regions) in which the grooves are not provided. The grooves 35 are elongated in the longitudinal direction of the diaper 1. In the upper-layer core 31, a plurality of grooves 35 that extend in the longitudinal direction are arranged at intervals in the width direction and also at intervals in the longitudinal direction. Also, the grooves 35 in the first embodiment are upward-facing grooves that are recessed from the skin-side surface of the upper-layer core 31 toward the non-skin side in the thickness direction.

The lower-layer core 32 also has grooves 36 in which portions of the lower-layer core 32 are recessed in the thickness direction. When viewed in the thickness direction, the lower-layer core 32 has groove regions A3 in which the grooves 36 are provided, and non-groove regions A4 in which the grooves 36 are not provided. However, contrary to the grooves 35 of the upper-layer core 31, the grooves 36 of the lower-layer core 32 are downward-facing grooves that are recessed from the non-skin-side surface of the lower-layer core 32 toward the skin side in the thickness direction. The grooves 36 are elongated in the longitudinal direction (predetermined direction) of the diaper 1. In the lower-layer core 32, a plurality of grooves 36 that extend in the longitudinal direction are arranged at intervals in the width direction and also at intervals in the longitudinal direction.

Also, the lower-layer core 32 has a central slit 37 (slit) that penetrates the lower-layer core 32 in the thickness direction, and a pair of side slits 38. The central slit 37 is a substantially rectangular slit that extends in the longitudinal direction, and is provided in the widthwise central portion of the crotch region of the diaper 1. The pair of side slits 38 are substantially rectangular slits that extend in the longitudinal direction. The pair of side slits 38 are provided at positions that are located on outward sides of the central slit 37 in the width direction and that are overlapped with at least a portion of the central slit 37 with respect to the longitudinal direction. The central slit 37 is longer in the width direction and the longitudinal direction than the side slits 38. Also, when viewed in the thickness direction, the entirety of the central slit 37 is overlapped with the upper-layer core 31, but the majority of the side slits 38 are not overlapped with the upper-layer core 31.

Note that the crotch region of the diaper 1 is a region where the crotch of the wearer is assumed to come into contact with the diaper 1. Specifically, the region below the joining portion 2 of the diaper 1 in the vertical direction (inward thereof in the longitudinal direction) is defined as the crotch region. Alternatively, the crotch region may be the central region when the unfolded and stretched diaper 1 (FIG. 2) is divided into three portions in the longitudinal direction.

Here, when the upper-layer core 31 and the lower-layer core 32 are each divided into two portions in the thickness direction, the portions located on the skin side are skin-side portions 311 and 321 and the portions located on the non-skin side are non-skin-side portions 312 and 322. Specifically, the skin-side portions 311 and 321 and the non-skin-side portions 312 and 322 are separated by a virtual line that extends horizontally from a center C in the thickness direction in the thickest portions of the non-groove regions A2 and A4.

In the upper-layer core 31, the average density of the super absorbent polymer 34 in the skin-side portions 311 of the groove regions A1 is lower than the average density of the super absorbent polymer 34 in the skin-side portions 311 of the non-groove regions A2. For this reason, the excreted fluid excreted on the skin-side surface of the upper-layer core 31 is more likely to flow in the groove regions A1, which are low SAP regions, than in the non-groove regions A2. Accordingly, the excreted fluid is dispersed in the longitudinal direction along the groove regions A1 that extend in the longitudinal direction.

In particular, the grooves 35 of the upper-layer core 31 are upward-facing grooves. Accordingly, the skin-side portion 311 in a groove region A1 is the space of a groove 35, and the average density of the super absorbent polymer 34 in the skin-side portion 311 of the groove region A1 is low (zero or close to zero). Because the upper-layer core 31 directly receives excreted fluid, by making the grooves 35 upward-facing grooves, the excreted fluid excreted on the skin-side surface of the upper-layer core 31 can immediately drop into the spaces of the grooves 35 and be dispersed in the longitudinal direction. Accordingly, even if a large amount of excreted fluid is excreted, it is possible to suppress the overflow of the excreted fluid on the skin-side surface of the upper-layer core 31.

Also, if the grooves 35 of the upper-layer core 31 are slits that penetrate in the thickness direction, the excreted fluid moves downward before dispersing along the groove regions A1, and the excreted fluid is locally absorbed in portions of the lower-layer core 32. There is a limit to the range over which the excreted fluid disperses from the local absorbed portions, and the excreted fluid cannot be absorbed over a wide range of the lower-layer core 32. In the upper-layer core 31 as well, excreted fluid moves to the lower-layer core 32 before being dispersed in the upper-layer core 31, and therefore the excreted fluid cannot be absorbed over a wide range of the upper-layer core 31.

To address this, the grooves 35 of the upper-layer core 31 of the present embodiment are grooves 35 (recessed portions) in which portions of the upper-layer core 31 are recessed in the thickness direction. For this reason, it is possible to prevent the excreted fluid from immediately moving from the groove regions A1 to the lower-layer core 32, and the excreted fluid can be dispersed along the groove regions A1 over a wide range of the upper-layer core 31.

Also, as shown in FIG. 6, some of the groove regions A1 of the upper-layer core 31 are provided in the region where the upper-layer core 31 and the lower-layer core 32 are overlapped with each other when viewed in the thickness direction. For this reason, the lower-layer core 32 can receive and absorb excreted fluid that has been dispersed along such groove regions A1 of the upper-layer core 31. Accordingly, the excreted fluid can be dispersed favorably in the lower-layer core 32 as well, and the excreted fluid is absorbed over a wide range of the lower-layer core 32. Also, after the lower-layer core 32 becomes saturated, the excreted fluid is dispersed and absorbed over a wide range of the upper-layer core 31.

Note that the lower-layer core 32 of the present embodiment has the central slit 37. In this case, it is preferable that groove regions A1 are provided in the portions of the upper-layer core 31 that are overlapped in the thickness direction with the portions of the lower-layer core 32 where the central slit 37 is not arranged. According to this configuration, the lower-layer core 32 can receive and absorb excreted fluid that has been dispersed along such groove regions A1.

Also, because the grooves 35 of the upper-layer core 31 of the present embodiment are different from slits, the lower-layer core 32 is not exposed to the skin side in the groove regions A1. For this reason, it is possible to prevent excreted fluid that has been absorbed in the portions of the lower-layer core 32 that face the groove regions A1 of the upper-layer core 31 from flowing back and touching the user's skin. Also, the skin-side portion 311 in each non-groove region A2 of the upper-layer core 31 is a high SAP region. For this reason, excreted fluid that has been absorbed by the skin-side portion 311 of the non-groove region A2 is reliably held by the super absorbent polymer 34. Accordingly, it is possible to prevent the rewetting of excreted fluid at the skin-side surface of the upper-layer core 31.

As described above, in the absorbent core 30 of the present embodiment, excreted fluid can be dispersed favorably in the upper-layer core 31 and the lower-layer core 32, and the absorption capacity is increased. For this reason, even if fluid is repeatedly excreted, the overflow or leakage of the excreted fluid can be suppressed. Also, the rewetting of excreted fluid can be suppressed.

Also, in FIG. 7, the bottom portions of the grooves 35 of the upper-layer core 31 are located at the boundary between the skin-side portion 311 and the non-skin-side portion 312, but there is no limitation to this, and the bottom portions of the grooves 35 may be at positions that are shallower or deeper than the boundary. Also, the high SAP region of the skin-side portions 311 of the non-groove regions A2 of the upper-layer core 31 may extend to the non-skin side beyond the bottom portions of the grooves 35 (or the ceiling portions of the grooves 35 if the grooves 35 are downward-facing grooves). Also, a portion of the non-skin-side portion 312 in a groove region A1 (i.e., a portion of the bottom portion of the groove 35 or a portion of the ceiling portion of the groove 35) may be a high SAP region. If a portion of the bottom portion (or ceiling portion) of a groove 35 is a high SAP region, excreted fluid is unlikely to be drawn in at the bottom portion (or ceiling portion) of the groove 35. For this reason, the excreted fluid is likely to disperse over the bottom portion of the groove 35 (or below the ceiling portion), and the ability of excreted fluid to disperse in the upper-layer core 31 is improved. This similarly applies to the lower-layer core 32 as well.

Furthermore, it is preferable that in the upper-layer core 31, the average density of the super absorbent polymer 34 in the non-skin-side portions 312 of the non-groove regions A2 is lower than the average density of the super absorbent polymer 34 in the skin-side portions 311 of the non-groove regions A2. According to this configuration, excreted fluid that has moved downward (toward the non-skin-side portions 312) from the skin-side portions 311 of the groove regions A1 is likely to move to the non-skin-side portions 312 of the non-groove regions A2, which are low SAP regions. For this reason, the ability of excreted fluid to disperse in the upper-layer core 31 is improved. For example, excreted fluid flows in the non-groove regions A2 that are arranged side-by-side with the groove regions A1 in the width direction, and disperses in the width direction as well. The excreted fluid that is further dispersed in the upper-layer core 31 is then received by the lower-layer core 32, and thus the excreted fluid is absorbed over a wider range of the lower-layer core 32. However, the present invention is not limited to the above configuration, and for example, the average density of the super absorbent polymer 34 in the non-skin-side portions 312 of the non-groove regions A2 may be equivalent to or greater than the average density of the super absorbent polymer 34 in the skin-side portions 311 of the non-groove regions A2.

Also, excreted fluid that has moved to the non-skin-side portions 312 of the non-groove regions A2 then moves to the lower-layer core 32 and is also sucked up by the skin-side portions 311 of the non-groove regions A2 as well. Because the skin-side portions 311 of the non-groove regions A2 are high SAP regions, the sucked-up excreted fluid is reliably held by the super absorbent polymer 34. Accordingly, it is possible to prevent the rewetting of excreted fluid at the skin-side surface of the upper-layer core 31.

If the grooves 35 of the upper-layer core 31 are upward-facing grooves, the super absorbent polymer 34 and the liquid absorbent fibers are arranged in the non-skin-side portions 312 of the groove regions A1. In this case, it is preferable that the average density of the super absorbent polymer 34 in the non-skin-side portions 312 of the groove regions A1 is lower than the average density of the super absorbent polymer 34 in the skin-side portions 311 of the non-groove regions A2.

According to this configuration, the excreted fluid that has flowed into the groove regions A1 (spaces) of the skin-side portions 311 of the upper-layer core 31 can easily move (move downward) to the non-skin-side portions 312 of the groove regions A1, which are low SAP regions. In other words, the liquid permeability in the groove regions A1 is improved. For this reason, even if excretion is repeated, it is possible to prevent the super absorbent polymer 34 that has absorbed the excreted fluid from inhibiting the liquid permeability of the groove regions A1.

Also, if the grooves 35 of the upper-layer core 31 are upward-facing grooves, it is preferable that the average density of the liquid absorbent fibers in the non-skin-side portions 312 of the non-groove regions A2 is higher than the average density of the liquid absorbent fibers in the non-skin-side portions 312 of the groove regions A1. According to this configuration, excreted fluid that has moved to the non-skin-side portions 312 of the groove regions A1 is likely to move to the non-skin-side portions 312 of the non-groove regions A2 due to the capillary effect. For this reason, the ability of excreted fluid to disperse in the upper-layer core 31 is improved. Also, excreted fluid dispersed in the upper-layer core 31 is received by the lower-layer core 32 and absorbed over a wider range thereof.

Also, the upper-layer core 31 and the lower-layer core 32 of the present embodiment respectively have thin fiber layers 313 and 323 at the skin-side surfaces thereof. The fiber layers 313 and 323 are constituted by the liquid absorbent fibers 33 and do not contain the super absorbent polymer 34. The fiber layers 313 and 323 facilitate the dispersion of excreted fluid in the planar direction at the skin-side surface of the upper-layer core 31 and the skin-side surface of the lower-layer core 32. Accordingly, the ability of excreted fluid to disperse in the upper-layer core 31 and the lower-layer core 32 is improved. However, a configuration is possible in which the upper-layer core 31 and/or the lower-layer core 32 do not have the fiber layers 313 and 323. Moreover, the thickness of the fiber layers 313 and 323 is low. For this reason, there is no influence on the difference in the average density of the super absorbent polymer 34 between the skin-side portions 311 and 321 (the portions that include the fiber layer 313 and the 323) and the non-skin-side portions 312 and 322 (the portions that do not include the fiber layers 313 and 323) of the upper-layer core 31 and the lower-layer core 32.

Also, although not shown, a thin fiber layer (a layer of liquid absorbent fibers 33 containing no super absorbent polymer 34) may be provided at the non-skin-side surfaces of the upper-layer core 31 and the lower-layer core 32. In this case as well, excreted fluid is likely to be dispersed in the planar direction at the non-skin-side surface of the upper-layer core 31 and the non-skin-side surface of the lower-layer core 32, further improving the ability of excreted fluid to disperse in the upper-layer core 31 and the lower-layer core 32.

Also, the comparison of the average density of the super absorbent polymer 34 and the comparison of the average density of the liquid absorbent fibers 33 in the portions of the absorbent core 30 can be performed by known methods. For example, the target absorbent core 30 is impregnated with liquid nitrogen, frozen, and then cut in the thickness direction with a razor, and cross-sections of the absorbent core 30 are acquired from the portions that are to be compared (e.g., the groove regions A1 and the non-groove regions A2). After allowing the absorbent core 30 to return room temperature, cross-sectional images are obtained with a magnification of 50 times using an electron microscope (e.g., VE7800 manufactured by Keyence Corporation). How high or low the average density of the super absorbent polymer 34 is in the target portions can be visually compared based on the cross-sectional images. Also, how high or low the average density of the liquid absorbent fibers 33 is in the target portions can also be visually compared based on the cross-sectional images. Furthermore, the number of super absorbent polymer 34 particles and the number of liquid absorbent fibers 33 per unit area may be counted in the cross-sectional image and compared.

As another example, the target absorbent core 30 is frozen and then cut to obtain cross sections of the absorbent core 30 from the portions that are to be compared. Then the average densities of the super absorbent polymer 34 may be compared based on images obtained by irradiating the cross sections with X-rays. The amount of X-ray transmission is lower in a portion where the super absorbent polymer 34 particles are overlapped a large amount. For this reason, in an X-ray image, a portion where the average density of the super absorbent polymer 34 is high is visually recognized as dark, and the target portions can be compared based on their lightness/darkness. Alternatively, the weight of the super absorbent polymer 34 and the weight of the liquid absorbent fiber 33 may be obtained by the measuring method described in Japanese Patent Application No. 2018-542962, and the weights may be converted to densities and then compared.

As described above, by forming the absorbent core 30 with a two-layer structure, the amount of excreted fluid that can be absorbed by the diaper 1 can be increased. However, if it is difficult for excreted fluid to move from the upper-layer core 31 to the lower-layer core 32, the excreted fluid may accumulate in spaces at the boundary between the upper-layer core 31 and the lower-layer core 32 in the thickness direction. In this case, the amount of excreted fluid that is absorbed by the lower-layer core 32 decreases, and the absorption performance of the lower-layer core 32 cannot be effectively utilized.

In view of this, in the lower-layer core 32 as well, it is preferable that the average density of the super absorbent polymer 34 in the skin-side portions 321 of the groove regions A3 is lower than the average density of the super absorbent polymer 34 in the skin-side portions 321 of the non-groove regions A4. Also, it is preferable that at least some of the groove regions A3 of the lower-layer core 32 are provided in the region where the upper-layer core 31 and the lower-layer core 32 are overlapped with each other when viewed in the thickness direction.

According to this configuration, excreted fluid that has moved to the boundary between the upper-layer core 31 and the lower-layer core 32 in the thickness direction is likely to be absorbed by the groove regions A3, which are low SAP regions, of the skin-side portions 321 of the lower-layer core 32. For this reason, the accumulation of excreted fluid at the boundary between the upper-layer core 31 and the lower-layer core 32 can be suppressed, and excreted fluid can be moved to the lower-layer core 32. Accordingly, the lower-layer core 32 can reliably absorb excreted fluid, and it is possible to prevent a reduction in the amount of the excreted fluid that can be absorbed by the diaper 1. On the other hand, the skin-side portions 321 of the non-groove regions A4 of the lower-layer core 32 are high SAP regions. For this reason, excreted fluid that has been absorbed by the skin-side portions 321 of the non-groove regions A4 is reliably held by the super absorbent polymer 34, and the rewetting of excreted fluid can be suppressed.

Also, the excreted fluid absorbed in the groove region A3 is likely to move to the spaces thereunder (non-skin-side portions 322). The excreted fluid is then dispersed in the longitudinal direction along the spaces of the groove regions A3. For this reason, the excreted fluid dispersed in the upper-layer core 31 is further dispersed in the lower-layer core 32, and the excreted fluid is absorbed over a wider range of the lower-layer core 32.

Also, the grooves 36 of the lower-layer core 32 are grooves 36 (recessed portions) in which portions of the lower-layer core 32 are recessed in the thickness direction. For this reason, unlike the case where the grooves 36 are slits that penetrate in the thickness direction, it is possible to prevent excreted fluid from accumulating in the grooves 36. In other words, excreted fluid can be dispersed even in the portions where the liquid absorbent fibers 33 and the super absorbent polymer 34 are arranged.

Also, if the grooves 36 of the lower-layer core 32 are downward-facing grooves, the ability of liquid to permeate downward (in the spaces) in the groove regions A3 is high. Accordingly, excreted fluid can be drawn to the non-skin-side portions 322 of the lower-layer core 32 and then dispersed, and the lower-layer core 32 can be effectively utilized. Also, the grooves 36 of the lower-layer core 32 are downward-facing grooves rather than slits, and because the spaces (recessed portions) of the grooves 36 are not present at the skin-side surface of the lower-layer core 32, the speed of movement of excreted fluid from the upper-layer core 31 to the lower-layer core 32 can be suppressed to an appropriate level. Accordingly, excreted fluid is dispersed in the upper-layer core 31 before moving to the lower-layer core 32.

Also, similarly to the upper-layer core 31, in the lower-layer core 32 as well, it is preferable that the average density of the super absorbent polymer 34 in the non-skin-side portions 322 of the non-groove regions A4 is lower than the average density of the super absorbent polymer 34 in the skin-side portions 321 of the non-groove regions A4.

According to this configuration, in the non-skin-side portions 322 of the lower-layer core 32, excreted fluid that has been dispersed in the longitudinal direction along the spaces of the grooves 36 is likely to move to the non-skin-side portions 322 of the non-groove regions A4, which are low SAP regions. For this reason, the ability of excreted fluid to disperse in the lower-layer core 32 is further improved. Also, excreted fluid that has moved to the non-skin-side portions 322 of the non-groove regions A4 is sucked up by the skin-side portions 321 of the non-groove regions A4. Because the skin-side portions 321 of the non-groove regions A4 are high SAP regions, the sucked-up excreted fluid is reliably held, and the rewetting of the excreted fluid can be suppressed.

Also, the planar shape of the lower-layer core 32 is slightly larger than the planar shape of the upper-layer core 31, and the lower-layer core 32 extends outward in the longitudinal direction from a longitudinal end 31a of the upper-layer core 31. In the portion of the lower-layer core 32 that extends outward in the longitudinal direction from the upper-layer core 31, excreted fluid does not accumulate at the boundary with the upper-layer core 31, but it is preferable that the groove regions A3 of the lower-layer core 32 are provided in this portion as well.

According to this configuration, excreted fluid can be dispersed to the region of the lower-layer core 32 that is not overlapped with the upper-layer core 31 in the thickness direction. Also, the longitudinal end portion of the lower-layer core 32 has flexibility due to the grooves 36 and can deform easily. Accordingly, the lower-layer core 32 can easily fit to the wearer's lower stomach region and buttocks region.

Also, it is preferable that groove regions A3 of the lower-layer core 32 have a portion overlapped with groove regions A1 of the upper-layer core 31 when viewed in the thickness direction. In the first embodiment, most of the grooves 35 of the upper-layer core 31 (excluding the two grooves 35A that overlap the central slit 37) are overlapped with the grooves 36 of the lower-layer core 32.

In the upper-layer core 31, a larger amount of excreted fluid flows downward (toward the non-skin side) through the groove regions A1 than through the non-groove regions A2. For this reason, if the groove regions A1 of the upper-layer core 31 and the groove regions A3 of the lower-layer core 32 are aligned, a large amount of excreted fluid that has passed through the groove regions A1 of the upper-layer core 31 can be received by the groove regions A3 of the lower-layer core 32, drawn into the lower-layer core 32 (the spaces of the non-skin-side portions 322), and dispersed. Accordingly, the accumulation of excreted fluid at the boundary between the upper-layer core 31 and the lower-layer core 32 can be further suppressed. Note that in the present embodiment, the grooves 35 of the upper-layer core 31 and the grooves 36 of the lower-layer core 32 overlapped therewith have the same shape and the same arrangement, but there is no limitation to this, and it is sufficient that they are at least partially overlapped.

Also, it is preferable that in at least a portion of the lower-layer core 32, the average density of the super absorbent polymer 34 in the skin-side portions 321 in a given region of the lower-layer core 32 is higher than the average density of the super absorbent polymer 34 in the non-skin-side portions 312 in a region of the upper-layer core 31 that faces the given region. According to this configuration, the speed of movement of the excreted fluid from the upper-layer core 31 to the lower-layer core 32 can be suppressed to an appropriate level. Accordingly, excreted fluid is dispersed in the upper-layer core 31 before moving to the lower-layer core 32.

Specifically, in the absorbent core 30 of the first embodiment, most of the groove regions A1 of the upper-layer core 31 are overlapped with the groove regions A3 of the lower-layer core 32, and most of the non-groove regions A2 of the upper-layer core 31 are overlapped with the non-groove regions A4 of the lower-layer core 32. As described above, the non-groove regions A4 of the skin-side portions 321 of the lower-layer core 32 are high SAP regions, and the non-groove regions A2 of the non-skin-side portions 312 of the upper-layer core 31 that face the non-groove regions A4 are low SAP regions, thus achieving the above-described relationship. On the other hand, the groove regions A3 of the skin-side portions 321 of the lower-layer core 32 and the groove regions A1 of the non-skin-side portions 312 of the upper-layer core 31 that face the groove regions A1 are both low SAP regions. For this reason, it is preferable that the average density of the super absorbent polymer 34 is higher in the groove regions A3 of the skin-side portion 321 of the lower-layer core 32 than in the groove regions A1 of the non-skin-side portions 312 of the upper-layer core 31.

Also, the lower-layer core 32 has the central slit 37 that extends in the longitudinal direction, in the widthwise central portion of the region that abuts the wearer's crotch region. For this reason, the lower-layer core 32 and the upper-layer core 31 that is overlapped therewith bend along the central slit 37 (along the longitudinal direction). Accordingly, the widthwise central portion of the absorbent core 30 deforms so as to protrude to the skin side relative to the side portions, and can easily fit to the wearer's crotch region.

Also, groove regions A1 of the upper-layer core 31 have portions (grooves 35A in FIG. 6) that are overlapped with the central slit 37 when viewed in the thickness direction. In the portion of the absorbent core 30 where the central slit 37 is provided, the ability to absorb excreted fluid is reduced, but excreted fluid can be dispersed in the groove regions A1 of the upper-layer core 31 that are overlapped with the central slit 37. In particular, it is preferable that longitudinal end portions of these groove regions A1 of the upper-layer core 31 extend outward in the longitudinal direction from the central slit 37. According to this configuration, excreted fluid can move to portions that are not overlapped with the central slit 37. Accordingly, the overflow of excreted fluid in the crotch region can be suppressed.

However, the present invention is not limited to the above configuration. The portion of the absorbent core 30 where the central slit 37 is provided has low stiffness. For this reason, a configuration is possible in which at least a portion of the central slit 37 of the lower-layer core 32 is not overlapped with groove regions A1 (grooves 35) of the upper-layer core 31 when viewed in the thickness direction. For example, as shown in the variation of FIG. 10A, a configuration is possible in which the entire region of the central slit 37 is not overlapped with the groove regions A1 of the upper-layer core 31. According to this configuration, it is possible to prevent the portion of the absorbent core 30 whose stiffness is low due to the central slit 37 from having an even lower stiffness due to the groove regions A1. And it is possible to suppress the loss of the shape of the absorbent core 30. Accordingly, the absorption performance of the absorbent core 30 is exhibited for a longer time. Also, excreted fluid is likely to pool in the central slit 37. For this reason, due to providing the non-groove region A2 as the region of the upper-layer core 31 that is overlapped with the central slit 37, the skin-side portions 311 are high SAP regions, and the movement of excreted fluid to the central slit 37 can be suppressed. Accordingly, the pooling of liquid in the central slit 37 can be suppressed.

Also, it is preferable that the grooves 35 of the upper-layer core 31 do not extend to the outer peripheral edge of the upper-layer core 31, and that the grooves 36 of the lower-layer core 32 do not extend to the outer peripheral edge of the lower-layer core 32. According to this configuration, it is possible to suppress the loss of the shape of the upper-layer core 31 and the lower-layer core 32. Also, it is possible to prevent excreted fluid from being dispersed to the outer peripheral edge of the upper-layer core 31 and the lower-layer core 32.

Also, as described above, the diaper 1 is provided with the crotch elastic members 17 that stretch and contract in the longitudinal direction in the region that abuts the wearer's crotch. As shown in FIG. 6, it is preferable that the central slit 37 and the groove regions A1 of the upper-layer core 31 have a portion that is overlapped with the crotch elastic members 17 when viewed in the thickness direction.

According to this configuration, deformation of the absorbent core 30 so as to protrude to the skin side due to the central slit 37 is promoted by the stretching and contracting of the crotch elastic members 17. Also, the absorbent core 30 is given flexibility due to the groove regions A1 and can easily deform to fit to the wearer's crotch. This deformation is also promoted by the stretching and contracting of the crotch elastic members 17. Accordingly, the fit of the diaper 1 to the wearer is improved.

Also, as shown in the variation of FIG. 10A, even in the case where the entire region of the central slit 37 of the lower-layer core 32 is not overlapped with the groove regions A1 of the upper-layer core 31, it is preferable that the central slit 37 has a portion overlapped with the crotch elastic members 17 when viewed in the thickness direction. According to this configuration, the deformation of the absorbent core 30 so as to protrude to the skin side due to the central slit 37 is promoted by the crotch elastic members 17, and a portion of the central slit 37 of the lower-layer core 31 bends so as to suppress the accumulation of excreted fluid.

Also, in the portion of the lower-layer core 32 that is overlapped with the central slit 37 with respect to the longitudinal direction, the widthwise side ends of the upper-layer core 31 are located outward of the central slit 37 in the width direction, and are located inward of widthwise sides ends of the lower-layer core 32 in the width direction. For this reason, the widthwise side portions of the lower-layer core 32 are likely to bend along the widthwise side ends of the upper-layer core 31 (along the longitudinal direction). In other words, the widthwise side portions of the lower-layer core 32 are likely to rise to the skin side.

Also, the lower-layer core 32 has the pair of side slits 38 on respective sides of the central slit 37 in the width direction. The positions of the side slits 38 in the width direction are substantially the same as the positions of the widthwise side ends of the upper-layer core 31. For this reason, the widthwise side portions of the lower-layer core 32 are likely to rise to the skin side due to the pair of side slits 38 as well. Accordingly, in the crotch region, the widthwise central portion of the absorbent core 30 is likely to deform so as to protrude to the skin side, the widthwise side portions are likely to rise to the skin side, and deformation into a W-shaped cross section is likely. For this reason, the diaper can more easily fit to the wearer's crotch.

Also, it is preferable that at least the grooves 35 of the upper-layer core 31 or the grooves 36 of the lower-layer core 32 are provided in a region where the upper-layer core 31 and the lower-layer core 32 are overlapped with each other outward of the central slit 37 of the lower-layer core 32 in the width direction. According to this configuration, the high-stiffness region where the two core layers 31 and 32 are overlapped with each other is given flexibility due to the grooves 35 and 36 and can easily deform, thus making it easier for the diaper to fit to the wearer's crotch.

Also, it is preferable that the groove regions A3 (grooves 36A) of the lower-layer core 32 are provided at positions that are outward in the width direction with respect to the side slits 38 of the lower-layer core 32 and that are at least partially overlapped with the side slits 38 with respect to the longitudinal direction. According to this configuration, the widthwise side portions of the lower-layer core 32 that have risen to the skin side have higher flexibility. For this reason, the widthwise side portions of the lower-layer core 32 can easily deform along the wearer's upper leg joints, and the fit is improved.

However, the present invention is not limited to the above configuration, and a configuration is possible in which the lower-layer core 31 does not have the central slit 37 and/or the side slits 38.

Also, it is preferable that the grooves 36 of the lower-layer core 32 are arranged so as to be separated from the outer peripheral edges of the central slit 37 and the side slits 38. According to this configuration, it is possible to suppress the loss of the shape of the lower-layer core 32.

Note that it is preferable that the widths (lengths in the width direction) of the central slit 37 and the side slits 38 are greater than the widths of the grooves 35 of the upper-layer core 31 and the grooves 36 of the lower-layer core 32 (the lengths of main grooves 351 and 361 in the width direction and the lengths of sub grooves 352 and 362 in the longitudinal direction). According to this configuration, the absorbent core 30 can easily deform into a W shape due to the central slit 37 and the side slits 38. On the other hand, by not making the widths of the grooves 35 and 36 too large, it is possible to prevent the absorbent core 30 from losing its shape. It is preferable that the widths of the central slit 37 and the side slits 38 are, for example, about 7 mm to 50 mm so that the absorption performance does not deteriorate too much. The width of the grooves 35 and 36 is preferably about 1 mm to 8 mm, for example. Also, the lengths of the central slit 37 and the side slits 38 in the longitudinal direction are lengths sufficient for abutting the wearer's crotch, such as about 50 to 300 mm, or preferably about 100 to 250 mm.

Also, the grooves 35 of the upper-layer core 31 and the grooves 36 of the lower-layer core 32 respectively include: main grooves 351 and 361 that are elongated in the longitudinal direction; and sub grooves 352 and 362 that extend in the width direction from the main grooves 351 and 361. Specifically, a plurality of sub grooves 352 and 362 extend in the width direction from the main grooves 351 and 361 at intervals in the longitudinal direction. A plurality of such main grooves 351 and 361 are arranged at intervals in the width direction and the longitudinal direction.

Due to the sub grooves 352 and 362, fluid excreted on the skin-side surface of the upper-layer core 31 and excreted fluid drawn into the non-skin-side portions 322 (spaces) of the lower-layer core 32 can be dispersed not only in the longitudinal direction but also in the width direction. Accordingly, the ability of excreted fluid to disperse in the upper-layer core 31 and the lower-layer core 32 can be further improved.

Also, by giving the absorbent core 30 a two-layer structure, the amount of excreted fluid that can be absorbed increases, but the absorbent core 30 becomes stiffer and can deform less easily. However, the upper-layer core 31 and the lower-layer core 32 of the present embodiment have the grooves 35 and 36. In particular, the grooves 35 and 36 are provided in the regions where the upper-layer core 31 and the lower-layer core 32 are overlapped with each and the stiffness is higher. For this reason, the absorbent core 30 is given flexibility and can easily deform along the wearer's body.

Also, the grooves 35 and 36 include not only the main grooves 351 and 361 that extend along the longitudinal direction, but also the sub grooves 352 and 362 that extend along the width direction. For this reason, the upper-layer core 31 and the lower-layer core 32 can not only easily bend along the longitudinal direction, but also easily bend along the width direction at the sub grooves 352 and 362. For this reason, the absorbent core 30 can easily curve in the longitudinal direction along the portion that extends from the wearer's lower stomach region to their buttocks region.

Also, in the first embodiment, most of the grooves 35 of the upper-layer core 31 are overlapped with the grooves 36 of the lower-layer core 32. Also, not only are the main grooves 351 of the upper-layer core 31 and the main grooves 361 of the lower-layer core 32 overlapped with each other, but also the sub grooves 352 of the upper-layer core 31 and the sub grooves 362 of the lower-layer core 32 are overlapped with each other. For this reason, the regions where the upper-layer core 31 and the lower-layer core 32 are overlapped with each other can even more easily bend in the longitudinal direction and the width direction along the grooves 35 and 36, and even more easily deform along the wearer's body.

Also, the longitudinal positions of sub grooves 352 and 362 that are adjacent to each other in the width direction are aligned with each other. For this reason, the upper-layer core 31 and the lower-layer core 32 can easily bend along the width direction at the sub grooves 352 and 362, and can easily deform along the wearer's body.

Also, as shown in FIG. 6, in the region backward in the longitudinal direction with respect to the central slit 37, the upper-layer core 31 and the lower-layer core 32 have regions A21, A41, and A42 in which the non-groove regions A2 and A4 are continuous from one end to the other end in the width direction. In this way, the regions A21, A41, and A42, which are continuous in the width direction and do not include the grooves 35 and 36, are provided in the back end portions of the upper-layer core 31 and the lower-layer core 32, and therefore the upper-layer core 31 and the lower-layer core 32 can easily bend in the longitudinal direction along such regions (at the longitudinal ends of the grooves 35 and 36). In other words, the bending of the upper-layer core 31 and the lower-layer core 32 in the longitudinal direction is not likely to be hindered by the grooves 35 and 36 that extend in the longitudinal direction. For this reason, the back end portions of the upper-layer core 31 and the lower-layer core 32 are likely to conform to the rounded buttocks region of the wearer.

Also, as shown in FIG. 6, a back end 32a of the lower-layer core 32 is located backward with respect to a back end 31a of the upper-layer core 31, and the back end 31a of the upper-layer core 31 is located in the non-groove region A42 that is continuous in the width direction of the lower-layer core 32. For this reason, the back end portion of the lower-layer core 32 can easily bend in the longitudinal direction along the back end 32a of the upper-layer core 31. Accordingly, the back end portion of the lower-layer core 32 can easily conform to the rounded buttocks region of the wearer.

Also, as described above, the back end portion of the absorbent core 30 can easily bend in the longitudinal direction, and thus W-shaped deformation in the crotch region of the absorbent core 30 (deformation due to the central slit 37 and the side slits 38) can be prevented from being transmitted to a position backward of the absorbent core 30. Accordingly, the back end portion of the absorbent core 30 can easily conform to the wearer's buttocks region.

Also, the region where the upper-layer core 31 and the lower-layer core 32 are not overlapped when viewed in the thickness direction (the region including only the lower-layer core 32) has low stiffness and high flexibility. For this reason, although not shown, in the region including only the lower-layer core 32, the width of the grooves 36 of the lower-layer core 32 (specifically, the length of the main grooves 361 in the width direction and the length of the sub grooves 362 in the longitudinal direction) may be smaller than in the region where the upper-layer core 31 and the lower-layer core 32 are overlapped with each other. According to this configuration, it is possible to suppress the loss of the shape of the lower-layer core 32.

Also, the boundary portion between the region where the upper-layer core 31 and the lower-layer core 32 are overlapped with each other and the region where they are not overlapped with each other when viewed in the thickness direction (the outer peripheral edge portion of the upper-layer core 31) can easily be a bending point of the absorbent core 30, but can also easily become a starting point of the loss of the shape of the absorbent core 30. In view of this, it is preferable that the groove regions A1 and A3 are not provided in the boundary portion, and it is particularly preferable that the groove regions A3 of the lower-layer core 32 are not provided. According to this configuration, it is possible to suppress the loss of the shape of the absorbent core 30.

As shown in FIGS. 8A and 8B, a core-wrapping sheet 39, which is a liquid-permeable sheet (e.g., tissue or nonwoven fabric), may be provided in the boundary portion between the upper-layer core 31 and the lower-layer core 32. One example of the core-wrapping sheet 39 is, as shown in FIG. 9A, a nonwoven fabric sheet that has been subjected to unevenness processing so as to have protruding portions 391 and recessed portions 392 that extend in the longitudinal direction. Due to the protrusions and recessions of the core-wrapping sheet 39, excreted fluid can be dispersed in the longitudinal direction, and the ability of excreted fluid to be dispersed in the upper-layer core 31 and the lower-layer core 32 is improved. Note that the protrusions and recessions of the core-wrapping sheet 39 are not limited to extending in the longitudinal direction, and may extend in the width direction or another direction. Also, the protrusions and recessions of the core-wrapping sheet 39 increase the amount of storage space for excreted fluid. Accordingly, even if a large amount of excreted fluid is excreted, the excreted fluid can be temporarily stored in the storage spaces of the core-wrapping sheet 39, thus making it possible to prevent excreted fluid from overflowing on the skin-side surface of the upper-layer core 31. Also, although not shown, in the core-wrapping sheet 39, the protruding portions 391 and the recessed portions 392 that extend in the longitudinal direction may be arranged intermittently in the longitudinal direction. In this case, the excreted fluid can also be dispersed in the width direction at the separating portions between the protrusions and recessions.

Also, by arranging the above core-wrapping sheet 39 at the boundary portion between the upper-layer core 31 and the lower-layer core 32 (by arranging the protruding and receding surface of the core-wrapping sheet 39 so as to face the non-skin-side surface of the upper-layer core 31), excreted fluid can be better dispersed at the non-skin-side surface of the upper-layer core 31. However, the core-wrapping sheet 39 is not limited to being a sheet that has protrusions and recessions.

Also, the core-wrapping sheet 39 is not limited to being arranged only at the boundary portion between the upper-layer core 31 and the lower-layer core 32. For example, as shown in FIG. 9B, the core-wrapping sheet 39 may be arranged so as to wrap around the lower-layer core 32 from the non-skin-side surface toward the skin-side surface side. According to this configuration, the loss of the shape of the lower-layer core 32 can be suppressed by the core-wrapping sheet 39. Also, the core-wrapping sheet 39 may be arranged at a portion other than the boundary portion between the upper-layer core 31 and the lower-layer core 32 (e.g., a portion of the lower-layer core 32 excluding the portion from p1 to p2 in FIG. 9B). According to this configuration, it is possible to eliminate the need for the adhesive that adheres the core-wrapping sheet 39 and the absorbent core 30 in the boundary portion between the upper-layer core 31 and the lower-layer core 32, and the ability of liquid to permeate from the upper-layer core 31 to the lower-layer core 32 is improved. Also, the core-wrapping sheet 39 may be arranged so as to wrap around the lower-layer core 32 from the skin-side surface to the non-skin-side surface side in the direction opposite to that in FIG. 9B. In this case, portions of the core-wrapping sheet 39 overlap each other if the lower-layer core 32 is to be completely surrounded, but because the overlapping region is arranged on the non-skin-side surface of the lower-layer core 32, liquid can move more smoothly from the upper-layer core 31 to the lower-layer core 32 than in the case where the overlapping region is arranged between the upper-layer core 31 and the lower-layer core 32, making it possible to suppress the accumulation of excreted fluid. Alternatively, the core-wrapping sheet 39 may be arranged in a portion of the upper-layer core 31 or in the periphery thereof, or the core-wrapping sheet 39 may not be arranged on the absorbent core 30.

Also, the shapes, number of, and arrangement positions of the grooves 35 of the upper-layer core 31 and the grooves 36 of the lower-layer core 32 are not limited to the examples illustrated in FIGS. 4 to 6. For example, in the absorbent core 30 of FIG. 10B, the grooves 35B and 36B provided in widthwise side portions of the front end portion of the upper-layer core 31 are inclined inward in the width direction toward the longitudinal center. For this reason, the front end portion of the absorbent core 30 that rises from the narrowest portion of the lower-layer core 32 can easily deform into an inverted triangular cup shape so as to wrap around the wearer's lower stomach region, and the fit of the diaper 1 is improved.

Also, for example, as shown in FIG. 11A, the sub grooves 352 and 362 extend from the main grooves 351 and 361 in the central portion of the absorbent core 30 in the longitudinal direction, but the sub grooves 352 and 362 are not provided in the end portions of the absorbent core 30 in the longitudinal direction. Also, the end portions of the lower-layer core 32 in the longitudinal direction are non-groove regions A4. As described above, the area ratio of the groove regions A1 and A3 may be smaller in the longitudinal end portions of the upper-layer core 31 and the lower-layer core 32 than in the central portion. According to this configuration, it is possible to suppress the loss of the shape of the absorbent core 30.

Also, for example, as shown in FIG. 11B, the grooves 35 and 36 may extend all the way to both longitudinal ends of the upper-layer core 31 and the lower-layer core 32. According to this configuration, excreted fluid can be dispersed a greater distance in the longitudinal direction. In this case, the non-groove regions A22 and A43 that are continuous in the width direction may be provided backward of the central slit 37 such that the back end portion of the absorbent core 30 conforms to the wearer's buttocks region (FIG. 11B), and although not shown, the grooves 35 and 36 may extend continuously from the longitudinal front ends to the longitudinal back ends of the upper-layer core 31 and the lower-layer core 32.

Also, the grooves 35 of the upper-layer core 31 and the grooves 36 of the lower-layer core 32 are not limited to having a predetermined length in the longitudinal direction, and may be grooves that extend in another direction such as the width direction. Furthermore, the grooves 35 of the upper-layer core 31 and the grooves 36 of the lower-layer core 32 may be grooves that extend in mutually different directions. Moreover, the grooves 35 of the upper-layer core 31 and the grooves 36 of the lower-layer core 32 may be provided so as to not overlap each other at all in the thickness direction. Moreover, the grooves 35 of the upper-layer core 31 and the grooves 36 of the lower-layer core 32 are not required to have the sub grooves 352 and 362. Also, a configuration is possible in which the groove regions A1 and A3 are not provided in the region where the upper-layer core 31 and the lower-layer core 32 are not overlapped with each other when viewed in the thickness direction.

### Second Embodiment

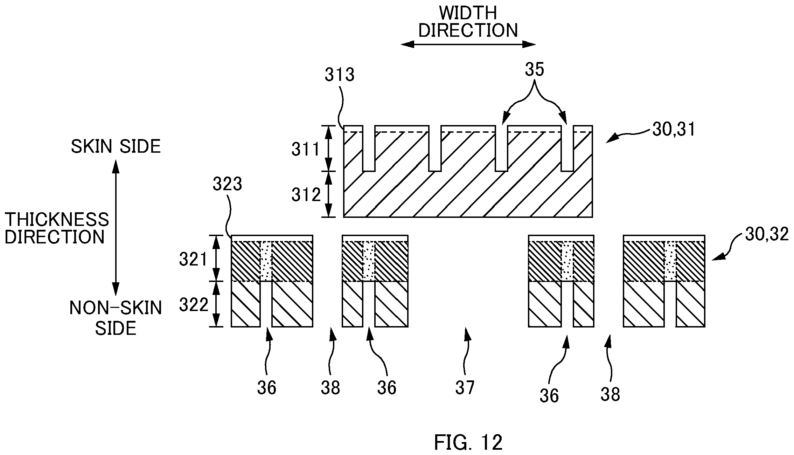

FIG. 12 is a schematic cross-sectional view of the absorbent core 30 according to a second embodiment. The lower-layer core 32 of the second embodiment is the same as the lower-layer core 32 of the first embodiment. Also, similarly to the upper-layer core 31 of the first embodiment, the upper-layer core 31 of the second embodiment has the grooves 35 that are recessed from the skin-side surface toward the non-skin side (upward-facing grooves). However, in the second embodiment, the super absorbent polymer 34 is uniformly distributed throughout the upper-layer core 31 (in the portion excluding the fiber layer 313). Specifically, the average density of the super absorbent polymer 34 is equivalent between portions of the upper-layer core 31, and the difference in such average density is smaller than the difference in the average density of the super absorbent polymer 34 between portions of the lower-layer core 32 (e.g., the groove regions A3 and the non-groove regions A4of the skin-side portion 321).

In this case, the excreted fluid excreted on the skin-side surface of the upper-layer core 31 can immediately drop into the spaces of the grooves 35 of the upper-layer core 31 and be dispersed. Since the super absorbent polymer 34 is uniformly distributed in the upper-layer core 31, even if excretion is repeated, the movement of liquid is less likely to be hindered by the super absorbent polymer 34 that has absorbed the excreted fluid, and the upper-layer core 31 has a good permeability of liquid. For this reason, the excreted fluid moves to the boundary between the upper-layer core 31 and the lower-layer core 32 in the thickness direction while being dispersing in the upper-layer core 31. The excreted fluid that has moved to the boundary is drawn into inside through the groove regions A3 of the skin-side portion 321 of the lower-layer core 32 (low SAP regions), and thereafter is further dispersed by the groove regions A3 of the non-skin-side portions 322 (spaces).

Thus, in the case where the upper-layer core 31 in which the super absorbent polymer 34 is uniformly distributed has the grooves 35, the grooves 35 may be upward-facing grooves shown in FIG. 12, and may be downward-facing grooves (not shown). In the case of downward-facing grooves, excreted fluid that has passed through the groove regions A1 of the upper-layer core 31 and moved to the non-skin-side portions 312 can be dispersed in the spaces of the grooves 35.

### Third Embodiment

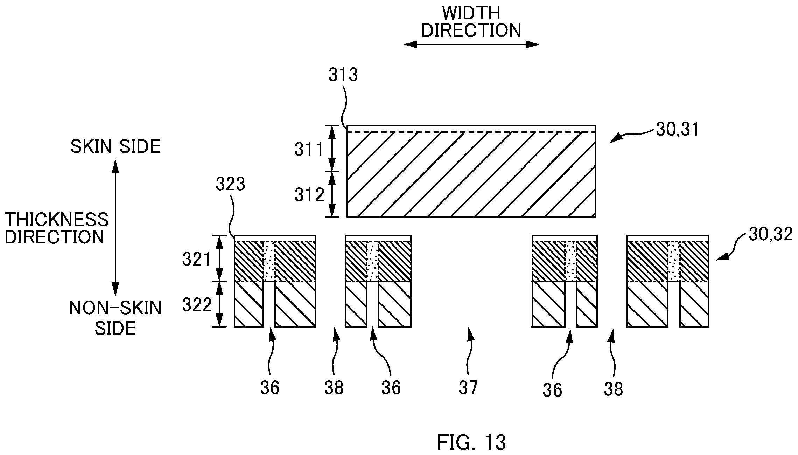

FIG. 13 is a schematic cross-sectional view of the absorbent core 30 according to a third embodiment. The lower-layer core 32 of the third embodiment is the same as the lower-layer core 32 of the first embodiment. Also, the upper-layer core 31 of the third embodiment does not have the grooves 35, and the super absorbent polymer 34 is uniformly distributed throughout the upper-layer core 31 (in the portion excluding the fiber layer 313).

In this case, the excreted fluid excreted on the skin-side surface of the upper-layer core 31 is taken into inside of the upper-layer core 31, and is moved to the boundary between the upper-layer core 31 and the lower-layer core 32 in the thickness direction while being dispersing in the planar direction. If the fiber layer 313 is provided on the skin-side surface of the upper-layer core 31, excreted fluid is dispersed in the planar direction on the skin-side surface of the upper-layer core 31. Since the super absorbent polymer 34 is uniformly distributed in the upper-layer core 31, even if excretion is repeated, the movement of liquid is less likely to be hindered by the super absorbent polymer 34 that has absorbed the excreted fluid, and the upper-layer core 31 has a good permeability of liquid. The excreted fluid that has moved to the boundary between the upper-layer core 31 and the lower-layer core 32 is drawn into inside through the groove regions A3 of the skin-side portion 321 of the lower-layer core 32 (low SAP regions), and thereafter is dispersed by the groove regions A3 of the non-skin-side portions 322 (spaces).

### Fourth Embodiment

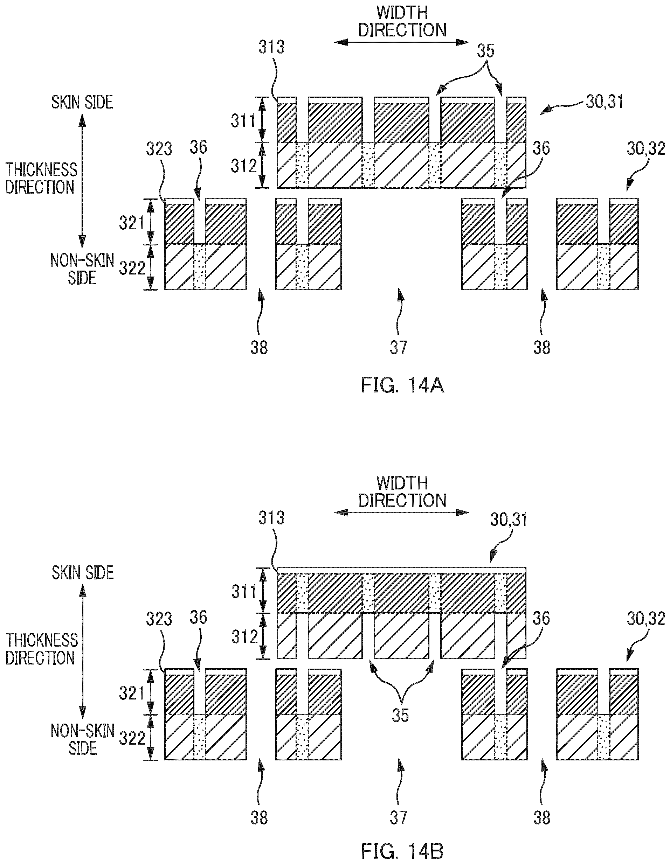

FIGS. 14A and 14B are schematic cross-sectional views of the absorbent core 30 according to a fourth embodiment. Contrary to the lower-layer core 32 of the first embodiment, the grooves 36 of the lower-layer core 32 of the fourth embodiment are upward-facing grooves. In this case as well, in the skin-side portions 321 of the lower-layer core 32, the groove regions A3 are the spaces and the average density of the super absorbent polymer 34 is lower in the groove regions A3 than in the non-groove regions A4. The excreted fluid that has passed through the upper-layer core 31 drops into the spaces of the grooves 36 of the skin-side portions 321 of the lower-layer core 32, and is dispersed in the longitudinal direction. This makes it possible to suppress the accumulation of the excreted fluid at the boundary between the upper-layer core 31 and the lower-layer core 32 in the thickness direction. It is possible to increase the ability to disperse excreted fluid in the lower-layer core.

If the grooves 36 of the lower-layer core 32 are upward-facing grooves, it is preferable that, similarly to the upper-layer core 31 of the first embodiment, the average density of the super absorbent polymer 34 in the non-skin-side portions 322 of the groove regions A3 is lower than the average density of the super absorbent polymer 34 in the skin-side portions 321 of the non-groove regions A4. This makes it possible to prevent the case where permeation of the groove regions A3 of the lower-layer core 32 is hindered by the super absorbent polymer 34 that has absorbed the excreted fluid. Also, it is preferable that the average density of the liquid absorbent fibers 33 in the non-skin-side portions 322 of the non-groove regions A4 of the lower-layer core 32 is higher than the average density of the liquid absorbent fibers 33 in the non-skin-side portions 322 of the groove regions A3. Accordingly, excreted fluid that has moved to the non-skin-side portions 322 of the groove regions A3 is more likely to move to the non-skin-side portions 322 of the non-groove regions A4 due to the capillary effect, further increasing the ability to disperse excreted fluid in the lower-layer core.

Even in the case where the grooves 36 of the lower-layer core 32 are upward-facing grooves, the grooves 35 of the upper-layer core 31 may be upward-facing grooves (FIG. 14A) or downward-facing grooves (FIG. 14B), or the upper-layer core 31 described in the second embodiment or the third embodiment may be used.

Also, as shown in FIG. 14B, due to aligning the positions of the grooves 35 (downward-facing grooves) of the upper-layer core 31 and the grooves 36 (upward-facing grooves) of the lower-layer core 32, a larger space can temporarily store excreted fluid that has passed through the skin-side portions 311 of the upper-layer core 31, and the stored excreted fluid can be dispersed along the grooves 35 and 36. For this reason, even if a large amount of fluid is excreted, it is possible to prevent the excreted fluid from overflowing on the skin-side surface of the upper-layer core 31.

Also, if the grooves 35 of the upper-layer core 31 are downward-facing grooves, the upper-layer core 31 can easily deform such that the central portion in the width direction protrudes toward the skin side relative to the side portions. Accordingly, deformation of the lower-layer core 32 due to the central slit 37 is promoted, and the absorbent core 30 can easily fit to the wearer's crotch region. On the other hand, if the grooves 36 of the lower-layer core 32 are upward-facing grooves, the lower-layer core 32 can easily deform such that the central portion in the width direction protrudes toward the non-skin side relative to the side portions. This therefore allows easier deformation along the rounded buttocks region.

### Fifth Embodiment

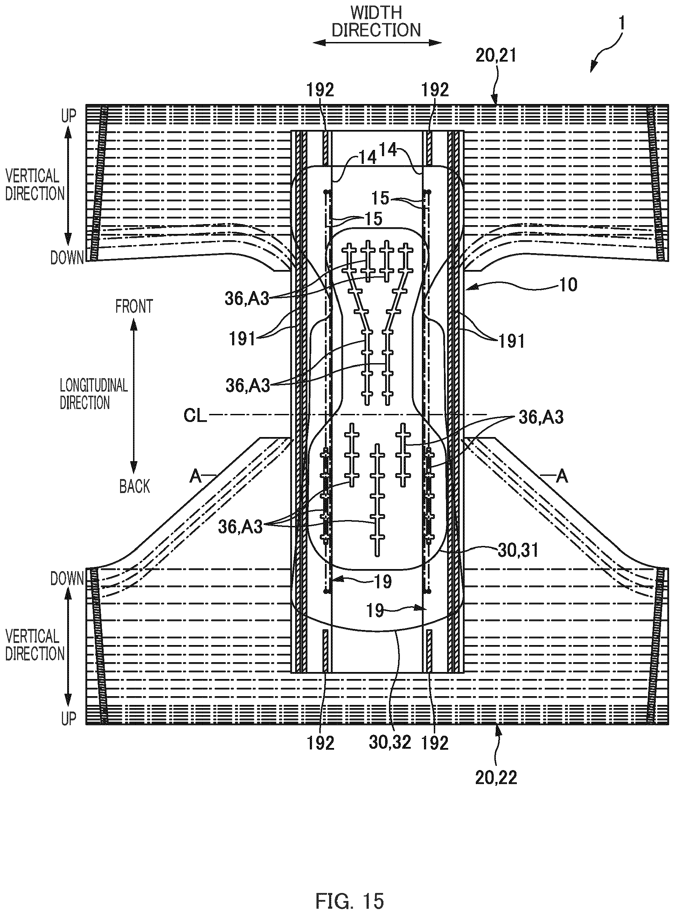

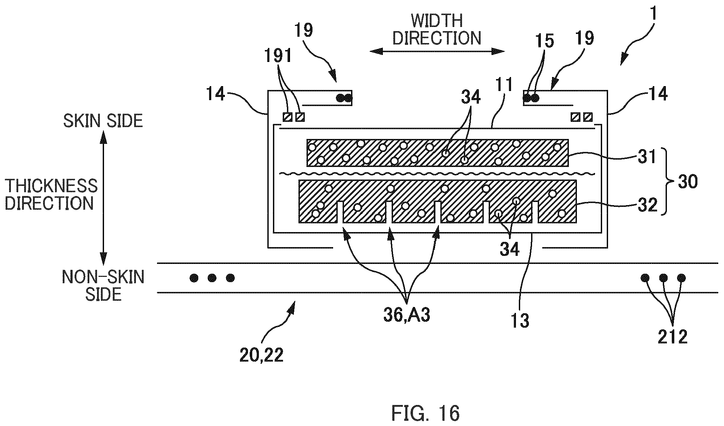

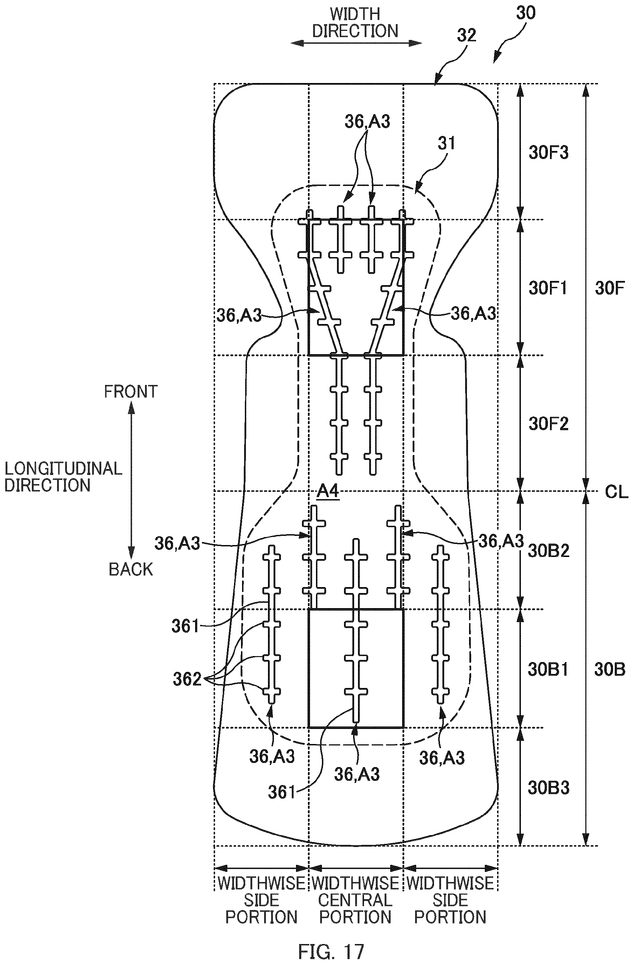

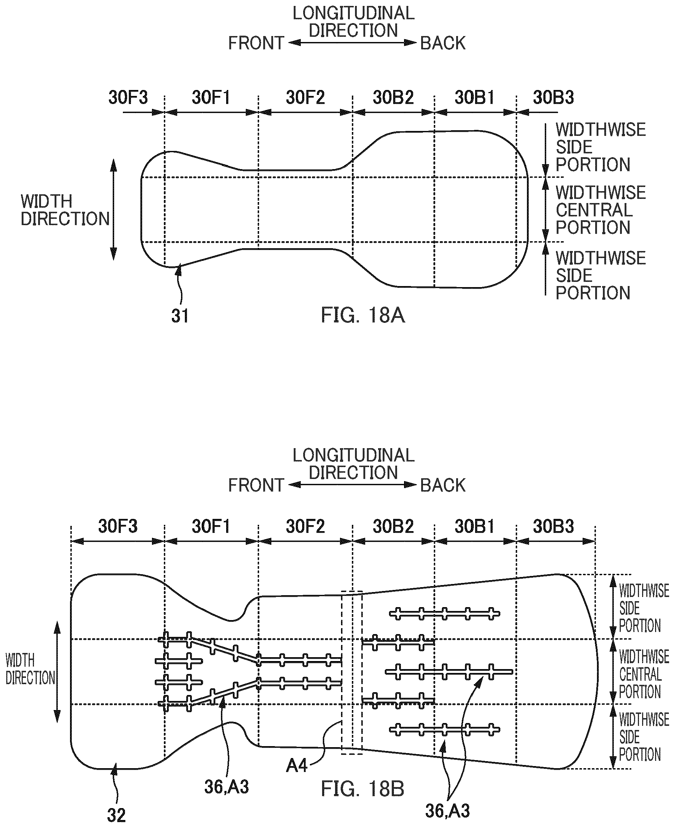

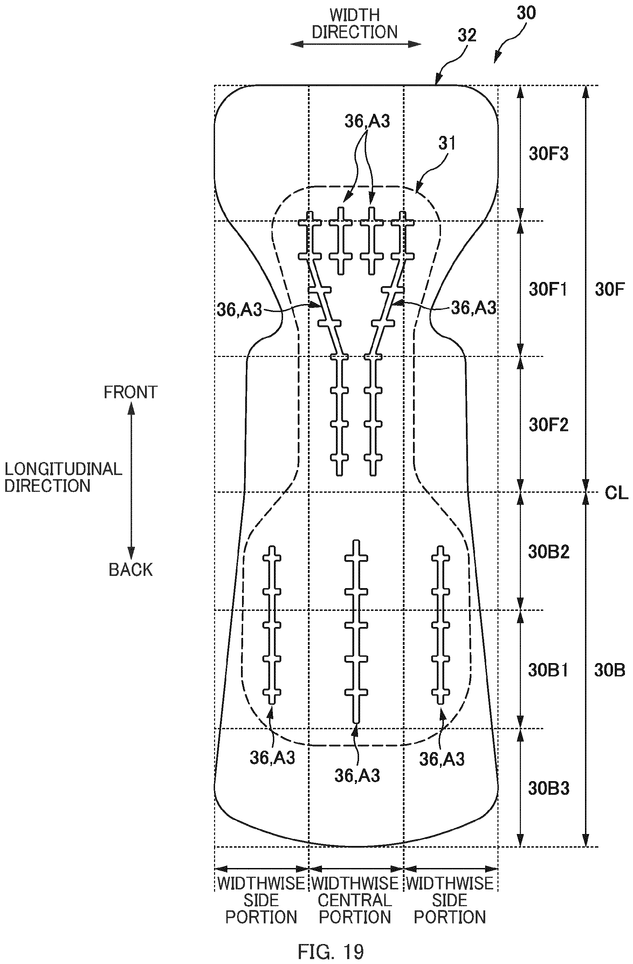

FIG. 15 is a schematic plan view of the diaper 1 according to a fifth embodiment in an unfolded and stretched state, as viewed from the skin side. FIG. 16 is a schematic cross-sectional view taken along A-A in FIG. 15. FIG. 17 is a plan view of the upper-layer core 31 and the lower-layer core 32 in an overlaid state as viewed from the skin side in the thickness direction. FIG. 18A is a plan view of the upper-layer core 31 as viewed from the skin side in the thickness direction. FIG. 18B is a plan view of the lower-layer core 32 as viewed from the skin side in the thickness direction. FIG. 19 is a plan view of the absorbent core 30 in a variation of the fifth embodiment, as viewed from the skin side in the thickness direction.

As shown in FIG. 16, the lower-layer core 32 of the fifth embodiment has grooves 36 (downward grooves) that are recessed from the non-skin-side surface of the lower-layer core 32 toward the skin side in the thickness direction, but does not have has a central slit 37 and a pair of side slits 38. However, the present invention is not limited to the above configuration, and a configuration is possible in which a slit (e.g., side slit 38) is provided in a region in the lower-layer core 32 where a groove region A3 is not provided.

As shown in FIG. 17, the absorbent core 30 has a "widthwise central portion" and a pair of "widthwise side portions", which are defined by dividing the maximum width of the absorbent core 30 into three portions (three equal portions) in the width direction. Also, the absorbent core 30 has a "front portion 30F" located on the front side in the longitudinal direction with respect to a product center line CL, and a "back portion 30B" located on the back side in the longitudinal direction with respect to the product center line CL.

The product center line CL (center line) is a line that divides the diaper 1 in the unfolded and stretched state (FIG. 15) into two portions (two equal portions) in the longitudinal direction. Also, the center of the absorbent core 30 in the longitudinal direction is not limited to being aligned with the product center line CL, and may deviate from the product center line CL. In other words, the absorbent core 30 may be biased toward the front side or the back side of the diaper 1. In this case, the front portion 30F and the back portion 30B have different lengths in the longitudinal direction.

The back portion 30B of the absorbent core 30 has regions obtained by dividing the maximum length (the length from the product center line CL to the backmost end of the absorbent core 30) into three portions (three equal portions) in the longitudinal direction. The central region obtained by such division will be called a "back first region 30B1", the region on the front side of the back first region 30B1 will be called a "back second region 30B2", and the region on the back side of the back first region 30B1 will be called a "back third region 30B3".

Similarly, the front portion 30F of the absorbent core 30 has regions obtained by dividing the maximum length (the length from the product center line CL to the frontmost end of the absorbent core 30) into three portions (three equal portions) in the longitudinal direction. The central region obtained by such division will be called a "front first region 30F1", the region on the back side of the front first region 30F1 will be called a "front second region 30F2", and the region on the front side of the front first region 30F1 will be called a "front third region 30F3".

In other words, in the following description, a rectangular region defined by the maximum width and maximum length of the absorbent core 10 is divided at the product center line CL into a front region and a back region in the longitudinal direction. The front region and the back region of the absorbent core 30 are also each divided into nine regions.

Note that in the fifth embodiment, the pull-on diaper 1 is illustrated as an example of an absorbent article, but the absorbent article according to the fifth embodiment is also applicable to another absorbent article such as a tape-type diaper or an absorbent pad. In the case of application to a tape-type diaper or an absorbent pad, the product center line is specified while the diaper is unfolded in the longitudinal direction, and the absorbent core is divided as shown in FIG. 17.

As described in the first embodiment, the average density of the super absorbent polymer 34 in the skin-side portions 321 of the groove regions A3 in the lower-layer core 32 is lower than the average density of the super absorbent polymer 34 in the skin-side portions 321 of the non-groove regions A4. For this reason, excreted fluid that has passed through the upper-layer core 31 is likely to be absorbed into the lower-layer core 32 from the groove regions A3, which are low SAP regions. The excreted fluid is then dispersed in the longitudinal direction along the spaces of the groove regions A3. For this reason, the accumulation of excreted fluid at the boundary between the upper-layer core 31 and the lower-layer core 32 can be suppressed. By excreted fluid moving smoothly from the upper-layer core 31 into the lower-layer core 32, it is possible to prevent the case where excreted fluid fails to be completely absorbed and flows beyond the surface of the diaper 1 and leaks to the outside. Also, by dispersing the excreted fluid, the lower-layer core 32 can be effectively utilized over a wide range in the surface direction.

In particular, in the case where the grooves 36 of the lower-layer core 32 are downward grooves, the ability of liquid to permeate downward (in the spaces) in the groove regions A3 is high. Accordingly, excreted fluid can be drawn to the non-skin-side portions of the lower-layer core 32 and then dispersed, and the lower-layer core 32 can be effectively utilized. Note that the grooves may be upward grooves as with the grooves 36 of the lower-layer core 32 in the fourth embodiment. In this case, while excreted fluid that has passed through the upper-layer core 31 drop into the space of the grooves 36 and is dispersed, excreted fluid can be absorbed into the lower-layer core 32 from three surfaces that forms each of the grooves 36 (bottom surface and a pair of side surfaces). Accordingly, the accumulation of excreted fluid at the boundary between the upper-layer core 31 and the lower-layer core 32 can be suppressed.

Furthermore, similarly to the absorbent core 30 shown in FIG. 7 in the first embodiment, it is preferable that the grooves 36 of the lower-layer core 32 are not grooves compressed in the thickness direction, and that the basis weight (g/m²) of the lower-layer core 32 (the liquid absorbent fibers 33 and the super-absorbent polymer 34) is lower in the groove regions A3 than in the non-groove region A4. According to this configuration, excreted fluid that has passed through the upper-layer core 31 is even more likely to flow into the groove regions A3 and be more easily absorbed into the lower-layer core 32 from the groove regions A3.

Note that the basis weights (g/m²) of the lower-layer core 32 in the groove regions A3 and in the non-groove region A42 can be compared using a known method. For example, the lower-layer core 32 is immersed in liquid nitrogen and frozen, and then cut in the thickness direction with a razor to obtain a cross-section of the lower-layer core 32 that includes the portions that are to be compared (the groove regions A3 and non-groove region A4). The lower-layer core 32 is then returned to room temperature, and an image of the cross-section is observed using an electron microscope to visually compare the quantities of material in the groove regions A3 and the non-groove region A4, or to count and compare the number of material layers.

Also, as an example of a method for manufacturing the lower-layer core 32 (absorbent core 30) in which the basis weight in the groove regions A3 is lower than in the non-groove region A4, the material constituting the lower-layer core 32 can be overlaid on a pattern plate whose bottom surface is provided with protrusions corresponding to the groove regions A3. Also, the basis weight and the average density of the material can be controlled by controlling the amount of material (liquid absorbent fibers 33 and super-absorbent polymer 34) supplied to the pattern plate.

As described above, excreted fluid that has passed through the upper-layer core 31 is even more likely to be absorbed into the lower-layer core 32 from the groove regions A3 of the lower-layer core 3. However, in many cases, the portions of the absorbent core 30 where the groove regions A3 are provided has thin thickness and low basis weight, and therefore rewetting to the surface of the diaper 1 is more likely to occur. Also, because the skin-side portion 321 of the lower-layer core 32 of the present embodiment is a low SAP region, rewetting from the lower-layer core 32 to the upper-layer core 31 is likely to occur, and there is a risk of causing rewetting to the surface of the diaper 1.

In view of this, in the absorbent core 30 of the fifth embodiment, a value obtained by dividing the area of the groove regions A3 of the lower-layer core 32 located in the widthwise central portion of the back first region 30B1 by the area of the widthwise central portion of the back first region 30B1 is set smaller than a value obtained by dividing the area of the groove regions A3 of the lower-layer core 32 located in the widthwise central portion of the front first region 30F1 by the area of the widthwise central portion of the front first region 30F1. Specifically, as shown in FIG. 17, only one main groove 361 that is longer in the longitudinal direction than in the width direction is provided in the widthwise central portion of the back first region 30B1, whereas four main grooves 361 are provided in the widthwise central portion of the front first region 30F1.

In the absorbent core 30, the widthwise central portion of the back first region 30B1 is a region likely to be subjected to body weight when the wearer is in a sitting posture or lying on their back. For this reason, by reducing the area ratio of the grooves 36 in the widthwise central portion of the back first region 30B1, even if a large amount of body weight is applied to the back first region 30B1, it is possible to reduce the amount of rewetting of excreted fluid from the groove regions A3.

On the other hand, in comparison with the back portion 30B, the front portion 30F of the absorbent core 30 is a region to come into contact with the wearer's excretion source and where excreted fluid is more likely to be excreted, and is a region less likely to be subjected to body weight when the wearer is in a sitting posture or the like. In particular, compared with the front second region 30F2 that comes into contact with the crotch portion of the wearer, and the front third region 30F3 that is likely to be in close contact with the wearer due to the waist elastic members 211, 221, and the like of the diaper 1, the front first region 30F1 therebetween is unlikely to be subjected to the body weight of the wearer. For this reason, by increasing the area ratio of the grooves 36 in the widthwise central portion of the front first region 30F1, a large amount of excreted fluid excreted by the wearer is less likely to accumulate at the boundary between the upper-layer core 31 and the lower-layer core 32 after passing through the upper-layer core 31, and is quickly drawn from the groove regions A3 into the lower-layer core 32. In particular, in the case where the wearer is a male, the front first region 30F1 is likely to come into contact with the wearer's excretion source, and thus the lower-layer core 32 can more effectively absorb excreted fluid. As a result, it is possible to prevent the case where excreted fluid fails to be completely absorbed by the upper-layer core 31 and flows beyond the surface of the diaper 1 and leaks to the outside. Also, rewetting is less likely to occur because body weight is less likely to be applied to the widthwise central portion of the front first region 30F1.

Also, it is preferable that the average basis weight (g/m²) of the lower-layer core 32 overall is higher than the average basis weight (g/m²) of the upper-layer core 31. In this case, the absorption and retention capacity of the upper-layer core 31 is relatively small. For this reason, by increasing the area ratio of the grooves 36 in the widthwise central portion of the front first region 30F1 as described above, a large amount of excreted fluid excreted from the excretion source can quickly move from the upper-layer core 31 into the lower-layer core 32. Accordingly, even if the average basis weight of the upper-layer core 31 is low, it is possible to effectively prevent the case where excreted fluid fails to be completely absorbed by the upper-layer core 31 and leaks to the outside.

However, the present invention is not limited to the above configuration, and a configuration is possible in which the average basis weight (g/m²) of the lower-layer core 32 overall is equal to or lower than the average basis weight (g/m²) of the upper-layer core 31. Note that the comparison of the average basis weight can be performed by known methods. For example, the basis weight is preferably obtained as follow: the upper-layer core 31 and the lower-layer core 32 are separated and taken out from the diaper 1, and then the weights (g) and the outer-shape areas (m²) of the cores 31 and 32 are measured, and the basis weights are calculated based thereon.

Also, the absorbent core 30 of the fifth embodiment also has a configuration in which, similarly to the absorbent core 30 in the embodiments described, the upper-layer core 31 and the lower-layer core 32 are overlaid on each other, and the groove regions A3 are at least partially provided in the overlapping portion including the upper-layer core 31 and the lower-layer core 32. In other words, the groove regions A3 are provided in a region where the upper-layer core 31 and the lower-layer core 32 are overlaid on each other, where the basis weight of the absorbent core 30 is relatively high, and where the retention of excreted fluid is high. For this reason, even if a large amount of excreted fluid is absorbed by the portions of the absorbent core 30 where the groove regions A3 are provided, rewetting from the groove regions A3 can be suppressed more than in the case of a single-layer absorbent core.

Note that FIG. 17 illustrates the absorbent core 30 in which the upper-layer core 31 has a shorter length in the width direction and the longitudinal direction than the lower-layer core 32. However, the present invention is not limited to this. For example, a configuration is possible in which the lower-layer core 32 have a smaller length in the width direction and the longitudinal direction than the upper-layer core 31, and a configuration is possible in which the length of the upper-layer core 31 in the width direction is longer (or shorter) than the length of the lower-layer core 32 in the width direction and the length of the upper-layer core 31 in the longitudinal direction is shorter (or longer) than the length of the lower-layer core 32 in the longitudinal direction. Also, in FIG. 17, the lower-layer core 32 extends farther than the upper-layer core 31 on both sides in the longitudinal direction. However, the present invention is not limited to this. For example, a configuration is possible in which the upper-layer core 31 extend farther than the lower-layer core 32 on both sides in the longitudinal direction, and a configuration is possible in which the lower-layer core 32 extends farther in the longitudinal direction than the upper-layer core 31 on one side in the longitudinal direction, and the upper-layer core 31 extends farther in the longitudinal direction than the lower-layer core 32 on the other side in the longitudinal direction.

Moreover, most of the excreted fluid that has not been absorbed by the absorbent core 30 at the portion in contact with the excretion source flows backward in the longitudinal direction through the widthwise central portion of the absorbent core 30. For this reason, it is preferable that the grooves 36 of the lower-layer core 32 are provided in the widthwise central portion of the back first region 30B1. However, this is a region where body weight is likely to be applied and rewetting is likely to occur. In view of this, as shown in FIG. 17, it is preferable that the portion of the lower-layer core 32 positioned in the widthwise central portion of the back first region 30B1 has a region provided with only one main groove 361 having a longer length in the longitudinal direction than in the width direction. In other words, it is preferable that there is a region that does not include a plurality of main grooves 361 arranged side by side in the width direction. According to this configuration, the excreted fluid flowing from the portion in contact with the excretion source to the back first region 30B1 passes through the upper-layer core 31 and then is dispersed in the longitudinal direction while being absorbed into the lower-layer core 32 by the groove regions A3 of the lower-layer core 32. Therefore, the excreted fluid flowing from the portion in contact with the excretion source can smoothly move from the upper-layer core 31 into the lower-layer core 32, making it possible to suppress the leakage of excreted fluid. Furthermore, the area ratio of the groove regions A1 can be kept small, and the amount of rewetting from the groove regions A1 can be reduced.

Unlike FIG. 17, a configuration is possible in which a plurality of main grooves 361 are arranged side by side in the width direction in a portion on the front side (a portion close to the portion in contact with the excretion source) of the widthwise central portion of the back first region 30B1. Also, the lower-layer core 32 located in the widthwise central portion of the back first region 30B1 may be provided with a groove 36 extending in a direction different from the longitudinal direction, or may be provided with no grooves 36 at all.

Also, known methods can be used to compare the area ratios of the groove regions A3 in the lower-layer core 32 and observe the number of groove regions A3. For example, in the case of separating or exposing the absorbent core 30 (the lower-layer core 32) from the diaper 1 and visually observing the groove regions A3, it is possible to first divide the absorbent core 30 as shown in FIG. 17, and then visually compare the area ratios of the groove regions A3 in the target portion and check the number of groove regions A1. Alternatively, it is possible to specify the groove regions A3 (outlines) in image data obtained by photographing the lower-layer core 32 from the thickness direction, and use area calculation software or the like to actually calculate the areas of the groove regions A3.

Also, as described above, the amount of X-ray transmission decreases in portions where the thickness of the super absorbent polymer 34 is high. In view of this, in the case where the density (basis weight) of the super absorbent polymer 34 in the groove regions A3 is smaller than that in the non-groove regions A4, the groove regions A3 can be specified based on an image obtained by irradiating the lower-layer core 32 with X-rays in the thickness direction. In other words, in the X-ray image, there is a difference in tone between the groove regions A3 and the non-groove regions A4. For this reason, it is possible to visually specify the groove regions A3 based on the shading of the X-ray image, and compare the area ratios of the groove regions A3 in the target portion as well as check the number of groove regions A3. Alternatively, it is possible to binarize the X-ray image, specify the groove regions A3 (outlines), and use area calculation software or the like to actually calculate the areas of the groove regions A3 and perform a comparison.

Moreover, most of the excreted fluid that has not been absorbed by the absorbent core 30 at the portion in contact with the excretion source flows backward in the longitudinal direction through the widthwise central portion of the absorbent core 30. In view of this, it is preferable that the average basis weight (g/m²) of the absorbent core 30 (the upper-layer core 31 and the lower-layer core 32) is higher in the widthwise central portion of the back first region 30B1 than in the pair of widthwise side portions of the back first region 30B1. Specifically, as shown in FIG. 17, the upper-layer core 31 and the lower-layer core 32 are not provided in portions of the widthwise side portions (rectangular regions) of the back first region 30B1, whereas the upper-layer core 31 and the lower-layer core 32 are provided in the entirety of the widthwise central portion (the entire region of the rectangular region).

According to this configuration, the portion of the absorbent core 30 in the widthwise central portion of the back first region 30B1 can absorb and retain a large amount of excreted fluid flowing from the portion in contact with the excretion source. Also, although the widthwise central portion of the back first region 30B1 is a portion where the body weight of the wearer is likely to be applied, rewetting can be suppressed.

Also, excreted fluid that has not been absorbed by the portion of the absorbent core 30 located in the widthwise central portion of the back first region 30B1 is likely to flow to the widthwise side portions. For this reason, portions of the lower-layer core 32 which are respectively located in the pair of widthwise side portions of the back first region 30B1 is preferably provided with the main groove 361. According to this configuration, the excreted fluid flowing to the widthwise side portions and having passed through the upper-layer core 31 is dispersed in the longitudinal direction while being absorbed into the lower-layer core 32 by the main grooves 361. In other words, in the widthwise side portions of the absorbent core 30, excreted fluid moves smoothly from the upper-layer core 31 into the lower-layer core 32, making it possible to suppress the leakage of excreted fluid, particularly lateral leakage.

Note that average basis weights of the absorbent core 30 can be compared using a known method. For example, the absorbent core 30 is separated from the diaper 1 and the absorbent core 30 is divided as shown in FIG. 17. After that, portions of the absorbent core 30 (the stack including the upper-layer core 31 and the lower-layer core 32) located in the widthwise central portion and the widthwise side portions of the back first region 30B1 are cut out, and the weight (g) of each portion is measured. Then the area a (m²) of the widthwise central portion of the back first region 30B1 is measured. Then, the basis weight (g/m²) is calculated by dividing the weights (g) of the absorbent core 30 located in the widthwise central portion and the widthwise side portions of the back first region 30B1 by the area a (m²).

Also, the planar shape of the upper-layer core 31 illustrated in FIG. 17 is an hourglass shape. The width of the upper-layer core 31 is narrowest in the front second region 30F2, and the width is wider on the front side and the back side in the longitudinal direction than in the front second region 30F2. The narrow portion (front second region 30F2) of the upper-layer core 31 is a portion that comes into contact with the excretion source or a portion close thereto. For this reason, it is preferable that the groove regions A3 of the lower-layer core 32 are provided in the widthwise side portions in the wide region on the back side, in the longitudinal direction, with respect to the narrow portion of the upper-layer core 31.

According to this configuration, similarly to the above description, excreted fluid flows backward from the portion in contact with the excretion source through the widthwise central portion of the absorbent core 30, and excreted fluid that has not been absorbed by the widthwise central portion of the absorbent core 30 flows to the widthwise side portions of the absorbent core 30. The excreted fluid passes through the upper-layer core 31, and then is absorbed into the lower-layer core 32 while being dispersed by the groove regions A3. In other words, in the widthwise side portions of the absorbent core 30, excreted fluid moves smoothly from the upper-layer core 31 into the lower-layer core 32, making it possible to suppress the leakage of excreted fluid, particularly lateral leakage.

Also, as shown in FIG. 18B, it is preferable that the lower-layer core 32 has a region in which the non-groove region A4 is continuous from one end to the other end in the width direction, between the groove regions A3 of the lower-layer core 32 located in the back second region 30B2 and the groove regions A3 of the lower-layer core 32 located in the front second region 30F2, with respect to the longitudinal direction.

According to this configuration, excreted fluid that has passed through the upper-layer core 31 is smoothly absorbed into the lower-layer core 32 by the groove regions A3 in the front second region 30F2 that is the portion in contact with the excretion source or a region close thereto. In the non-groove region A2 on the back side of the front second region 30F2, that is to say in the region where the basis weight of the lower-layer core 32 is high, the aforementioned excreted fluid is reliably absorbed and retained, and rewetting can be suppressed. Also, excreted fluid that has not been completely absorbed by the portion of the absorbent core 30 located in the front second region 30F2 and has flowed to the back side passes through of the back second region 30B2, and then is absorbed into the lower-layer core 32 while being dispersed by the groove regions A3. This makes it possible to suppress the leakage of excreted fluid.

Also, it is preferable that a value obtained by dividing the area of the groove regions A3 of the lower-layer core 32 that are located in the widthwise central portion of the back second region 30B2 by the area of the widthwise central portion of the back second region 30B2 is greater than a value obtained by dividing the area of the groove regions A3 of the lower-layer core 32 that are located in the widthwise central portion of the back first region 30B1 by the area of the widthwise central portion of the back first region 30B1. Specifically, only one main groove 361 of the lower-layer core 32 that is longer in the longitudinal direction than in the width direction is provided in the widthwise central portion of the back first region 30B1, whereas three main grooves 361 of the lower-layer core 32 are provided in the widthwise central portion of the back second region 30B2.

By increasing the area ratio of the groove regions A3 in the back second region 30B2 that is close to the region in contact with the excretion source, a large amount of excreted fluid flowing from the region in contact with the excretion source and having passed through the upper-layer core 31 can be smoothly moved into the lower-layer core 32, and the leakage of excreted fluid can be suppressed. On the other hand, by reducing the area ratio of the groove regions A3 in the back first region 30B1 that is likely to be subjected to the wearer's body weight, rewetting can be suppressed.

However, the present invention is not limited to the above configuration, and as in a variation shown in FIG. 19, a configuration is possible in which the widthwise central portion of the back second region 30B2 is provided with the same number of (here, one) main grooves 361 as the widthwise central portion of the back first region 30B1. For example, in the case of a wearer who excretes a large amount of excreted fluid, as shown in FIG. 17, it is preferable to provide many groove regions A3 in the portion of the lower-layer core 32 located in the widthwise central portion of the back second region 30B2. On the other hand, in the case of a wearer who excretes a small amount of excreted fluid, the leakage of excreted fluid can be suppressed even if few groove regions A3 of the lower-layer core 32 are located in the widthwise central portion of the back second region 30B2 as shown in FIG. 19.

Also, it is preferable that the number of main grooves 361 that are longer in the longitudinal direction than in the width direction is greater in the portion of the lower-layer core 32 located in the widthwise central portion of the front first region 30F1 than in the portion of the lower-layer core 32 located in the widthwise central portion of the front second region 30F2. In the lower-layer core 32 in FIG. 17, two main grooves 361 are provided in the front second region 30F2, whereas four main grooves 361 are provided in the front first region 30F1. It is preferable that the area ratio of the groove regions A3 of the lower-layer core 32 is larger in the front first region 30F1 than in the front second region 30F2.

As described above, the body weight of the wearer is less likely to be applied to the front first region 30F1 than to the front second region 30F2, which comes into contact with the crotch portion of the wearer. For this reason, the rewetting of excreted fluid can be suppressed even if the number of main grooves 361 is increased. Also, in the case where the wearer is a male, the region relatively on the front side is the portion that comes into contact with the excretion source. For this reason, by increasing the number of main grooves 361 in the front first region 30F1, a large amount of excreted fluid excreted from the excretion source pass through the upper-layer core 31 and then is dispersed while being quickly absorbed into the lower-layer core 32 by the large number of main grooves 361. Therefore, the leakage of excreted fluid can be suppressed.

Also, in the widthwise central portion of the back first region 30B1 and the widthwise central portion of the back side second region 30B2, a main groove 361 of the lower-layer core 32 is provided on the center line that divides the rectangular regions into two portions in the width direction. According to this configuration, excreted fluid that has flowed backward from the portion in contact with the excretion source through the widthwise central portion of the absorbent core 30 and has passed through the upper-layer core 31 is likely to flow into the groove regions A3 of the lower-layer core 32 and is easily absorbed into the lower-layer core 32.

On the other hand, in the widthwise central portion of the front first region 30F1 and the widthwise central portion of the front second region 30F2, a main groove 361 of the lower-layer core 32 is not provided on the center line that divides the rectangular regions into two portions in the widthwise direction, but main grooves 361 are provided on both sides of the center line. According to this configuration, the excretion source of the wearer is less likely to be located at a main groove 351 of the lower-layer core 32, and a sufficient basis weight is ensured for the absorbent core 30 in the region that receives the greatest amount of excreted fluid. For this reason, rewetting can be effectively suppressed.

Also, as shown in FIG. 15, the diaper 1 has a pair of leak-proof wall portions 19 that can rise to the skin side, in two widthwise side portions of the absorbent core 30 (absorbent main body 10). Specifically, as shown in FIG. 16, the side sheets 14 are folded back from the widthwise side portions of the non-skin-side surface of the back sheet 13 toward the widthwise side portions of the skin-side surface of the top face sheet 11. Leak-proof-wall elastic members 15 are fixed, while being stretched in the longitudinal direction, to the inward widthwise end portions of the folded side sheets 14.

Also, as shown in FIG. 15, the side sheets 14 are joined to the skin-side surface of the top face sheet 11 by first joining portions 191 and second joining portions 192 to which an adhesive or the like has been applied. The first joining portions 191 extend from the longitudinal one end to the longitudinal other end of the absorbent main body 10. The second joining portions 192 are provided in the two longitudinal end portions of the absorbent main body 10, at locations inward of the first joining portions 191 in the width direction. Accordingly, in the side sheets 14, the portions that are inward of, in the width direction, the widthwise inner ends (rising support points) of the first joining portions 191, and are inward of, in the longitudinal direction, the longitudinal inner ends (rising support points) of the second joining portions 192 can rise to the skin side, and such portions are the leak-proof wall portions 19.

In the case where the diaper 1 has the leak-proof wall portions 19, it is preferable that the lower-layer core 32 has the groove regions A3 that are at least partially overlapped with the leak-proof wall portions 19 when the diaper 1 in the unfolded and stretched state (FIG. 15) is viewed in the thickness direction. According to this configuration, excreted fluid that has been blocked by the leak-proof wall portions 19 and has been absorbed by the upper-layer core 31 can be rapidly absorbed from the groove regions A3 into the lower-layer core 32, and it is possible to suppress the case where excreted fluid flows beyond the leak-proof wall portions 19 and leaks.

Also, in the regions where the leak-proof wall portions 19 is provided, the wearer's skin comes into contact with the side sheets 14 instead of coming into direct contact with the top face sheet 11. For this reason, even if rewetting occurs due to excreted fluid from the groove regions A3 that are overlapped with the leak-proof wall portions 19 in the thickness direction, the wearer is not likely to feel such rewetting, and a reduction in the comfort of the diaper 1 can be prevented. Also, the leak-proof wall portions 19 rising to the skin side due to contraction of the leak-proof-wall elastic members 15 are likely to function as cushioning material, and the body weight of the wearer is not likely to be applied to the portions where the leak-proof wall portions 19 are provided. For this reason, rewetting can be suppressed even if the groove regions A3 are provided in portions that are overlapped with the leak-proof wall portions 19 in the thickness direction.

Also, as shown in FIG. 16, in the case where the upper-layer core 31 also contains the super absorbent polymer 34 similarly to the lower-layer core 32, it is preferable that the average density of the super absorbent polymer 34 in the upper-layer core 31 overall is higher than the average density of the super absorbent polymer 34 in the lower-layer core 32 overall.

Compared with excreted fluid existing between liquid absorbent fibers, excreted fluid retained in the super absorbent polymer 34 is less likely to move freely, and is less likely to cause rewetting. For this reason, by raising the average density of the super absorbent polymer 34 in the upper-layer core 31, excreted fluid that has flowed back from the lower-layer core 32 (in particular, the groove regions A3) to the upper-layer core 31 can be reliably retained by the super absorbent polymer 34 in the upper-layer core 31. For this reason, even if the absorbent core 30 is subjected to the body weight of the wearer, it is possible to suppress rewetting from the upper-layer core 31 to the top face sheet 11.

Also, although the upper-layer core 31 of the fifth embodiment does not have the groove regions A1 (grooves 35), the present invention is not limited to this. For example, the upper-layer core 31 may have grooves 35 that are recessed from the skin-side surface of the upper-layer core 31 toward the non-skin side in the thickness direction (upward grooves), or may have grooves 36 that are recessed from the non-skin-side surface of the upper-layer core 31 toward the skin side in the thickness direction (downward grooves) .

In the case where the upper-layer core 31 has the groove regions A1, similarly to the lower-layer core 32, it is preferable that the area ratio of the groove regions A1 of the upper-layer core 31 located in the widthwise central portion of the back first region 30B1 is relatively small. For example, it is preferably smaller than the area ratio of the groove regions A1 of the upper-layer core 31 located in the widthwise central portion of the front first region 30F1.

By providing the groove regions A1 in the upper-layer core 31 located in the widthwise central portion of the front first region 30F1, excreted fluid that has flowed from the portion in contact with the excretion source through the widthwise central portion of the absorbent core 30 to the back first region 30B1 can be absorbed into the absorbent core 30 by the groove regions A1 in the upper-layer core 31. Accordingly, the leakage of excreted fluid can be suppressed. However, by reducing the area ratio of the groove regions A1, it is possible to suppress rewetting of the excreted fluid.

On the other hand, the front first region 30F1 is a portion that comes into contact with the excretion source or a portion close thereto. For this reason, by increasing the area ratio of the groove regions A1 of the upper-layer core 31 in the widthwise central portion of the front first region 30F1, a large amount of excreted fluid excreted by the wearer can be rapidly absorbed into the absorbent core 30 by the groove regions A1 in the upper-layer core 31, making it possible to suppress the leakage of excreted fluid.

Note that when the absorbent core 30 is viewed in the thickness direction, the positions of the groove regions A1 of the upper-layer core 31 and the groove regions A3 of the lower-layer core 32 may be aligned with each other or may be shifted from each other. If the positions are aligned with each other, excreted fluid absorbed from the groove regions A1 of the upper-layer core 31 can be quickly absorbed into the lower-layer core 32 and dispersed by the groove regions A3 of the lower-layer core 32. On the other hand, in the case where the positions of the groove regions A1 and A3 are shifted from each other, it is possible to suppress a local decrease in the basis weight of portions of the absorbent core 30 where the groove regions A1 and A3 are provided, and rewetting from the groove regions A1 and A3 can be suppressed.

### Other embodiments

Although the embodiments of the present disclosure have been described hereinabove, the above embodiments of the present disclosure are simply to facilitate understanding of the present disclosure and are not in any way to be construed as limiting the present disclosure. The present disclosure may variously be changed or altered without departing from its gist and encompass equivalents thereof.

For example, the upper-layer core 31 is not limited to being an absorbent core obtained by molding liquid absorbent fibers 33 containing a super absorbent polymer 34 into a predetermined shape. The upper-layer core 31 may be an SAP sheet in which an SAP layer is adhered to a hydrophilic sheet, an air-laid sheet in which liquid absorbent fibers are formed into a sheet by an air-laid method, or the like.

### Reference Signs

1 (pull-on) diaper (absorbent article), 2 joining portion,
10 absorbent main body, 11 top-face sheet, 12 intermediate sheet,
13 back sheet,
14 side sheet, 15 leak-proof-wall elastic member, 16 leg elastic member,
17 crotch elastic member (elastic member), 18 cover sheet,
20 exterior member, 21 front waist portion, 22 back waist portion,
23 crotch portion,
211,221 waist elastic member, 212,222 leg elastic member,
30 absorbent core, 31 upper-layer core, 32 lower-layer core,
33 liquid absorbent fiber, 34 super absorbent polymer,
35 groove (second groove), 36 groove,
37 central slit (slit), 38 side slit,
39 core-wrapping sheet,
A1 groove region (second groove region), A2 non-groove region (second non-groove region),
A3 groove region, A4 non-groove region

## Claims

1. An absorbent article having a longitudinal direction, a width direction, and a thickness direction,
the absorbent article comprising: an absorbent core,
the absorbent core including an upper-layer core and a lower-layer core,
the lower-layer core being arranged on a non-skin side in the thickness direction with respect to the upper-layer core,
the lower-layer core including a super absorbent polymer,
the lower-layer core having a groove in which a portion of the lower-layer core is recessed in the thickness direction,
the groove being elongated in a predetermined direction,
when viewed in the thickness direction, the lower-layer core having
a groove region in which the groove is provided and
a non-groove region in which the groove is not provided,
when viewed in the thickness direction, at least a portion of the groove region being provided in a region where the upper-layer core and the lower-layer core are overlapped with each other,
when the lower-layer core is divided into two portions in the thickness direction,
an average density of the super absorbent polymer in a skin-side portion of the groove region being lower than an average density of the super absorbent polymer in a skin-side portion of the non-groove region,
the skin-side portion being a portion located on a skin side,
the non-skin-side portion being a portion located on a non-skin side.

2. An absorbent article according to claim 1, wherein
the absorbent core has a widthwise central portion and a pair of widthwise side portions,
the widthwise central portion and the pair of widthwise side portions being defined by dividing a maximum width of the absorbent core into three portions in the width direction,
the absorbent core has
a front portion located on a front side in the longitudinal direction with respect to a product center line and
a back portion located on a back side in the longitudinal direction with respect to the product center line,
the product center line being a line that divides the absorbent article in the unfolded and stretched state into two portions in the longitudinal direction,
the back portion has a back first region that is a central region obtained by dividing a maximum length of the back portion into three portions in the longitudinal direction,
the front portion has a front first region that is a central region obtained by dividing a maximum length of the front portion into three portions in the longitudinal direction,
concerning a value obtained by dividing an area of the groove region of the lower-layer core located in the widthwise central portion in the back first region by an area of the widthwise central portion in the back first region,
concerning a value obtained by dividing an area of the groove region of the lower-layer core located in the widthwise central portion in the front first region by an area of the widthwise central portion in the front first region,
the former value is smaller than the latter value.

3. An absorbent article according to claim 2, wherein
a portion of the lower-layer core located in the widthwise central portion in the back first region has a region provided with only one groove that is elongated in the longitudinal direction,
the longitudinal direction being the predetermined direction.

4. An absorbent article according to claim 2 or 3, wherein
an average basis weight of the absorbent core is higher in the widthwise central portion in the back first region than in the pair of widthwise side portions in the back first region, and
each of portions of the lower-layer core respectively located in the pair of widthwise side portions in the back first region has a groove that is elongated in the longitudinal direction,
the longitudinal direction being the predetermined direction.

5. An absorbent article according to any one of claims 2 to 4, wherein
the back portion has a back second region on the front side in the longitudinal direction with respect to the back first region,
the front portion has a front second region on the back side in the longitudinal direction with respect to the front first region, and
the lower-layer core has a region in which the non-groove region is continuous from one end to another end in the width direction, between the groove region of the lower-layer core located in the back second region and the groove region of the lower-layer core located in the front second region, with respect to the longitudinal direction.

6. An absorbent article according to any one of claims 2 to 5, wherein
the back portion has a back second region on the front side in the longitudinal direction with respect to the back first region, and
concerning a value obtained by dividing an area of the groove region of the lower-layer core located in the widthwise central portion in the back second region by an area of the widthwise central portion in the back second region,
concerning the value obtained by dividing the area of the groove region of the lower-layer core located in the widthwise central portion in the back first region by the area of the widthwise central portion in the back first region,
the former value is greater than the latter value.

7. An absorbent article according to any one of claims 2 to 6, wherein
the front portion has a front second region on the back side in the longitudinal direction with respect to the front first region, and
a portion of the lower-layer core located in the widthwise central portion in the front first region has more number of grooves that are elongated in the longitudinal direction than a portion of the lower-layer core located in the widthwise central portion in the front second region,
the longitudinal direction being the predetermined direction.

8. An absorbent article according to any one of claims 2 to 7, wherein
an average basis weight of the lower-layer core is higher than an average basis weight of the upper-layer core.

9. An absorbent article according to any one of claims 1 to 8, wherein
the absorbent article further comprises a pair of leak-proof wall portions in two widthwise side portions of the absorbent core,
the pair of leak-proof wall portions being capable of rising to the skin side, and
the lower-layer core has the groove region that is at least partially overlapped with the leak-proof wall portion when the absorbent article in an unfolded and stretched state is viewed in the thickness direction.

10. An absorbent article according to any one of claims 1 to 9, wherein
the upper-layer core includes a super absorbent polymer, and
an average density of the super absorbent polymer in the upper-layer core is higher than an average density of the super absorbent polymer in the lower-layer core.

11. An absorbent article according to claim 1, wherein
an average density of the super absorbent polymer in a non-skin-side portion of the non-groove region is lower than
the average density of the super absorbent polymer in the skin-side portion of the non-groove region.

12. An absorbent article according to claim 1 or 2, wherein
the groove is a groove that is recessed from a non-skin-side surface of the lower-layer core toward the skin side in the thickness direction.

13. An absorbent article according to any one of claims 1 to 3, wherein
the upper-layer core includes a super absorbent polymer, and
when the upper-layer core is divided into two portions in the thickness direction,
in at least a portion of the lower-layer core, an average density of the super absorbent polymer in the skin-side portion in a given region of the lower-layer core is higher than an average density of the super absorbent polymer in a non-skin-side portion in a region of the upper-layer core that faces the given region, the skin-side portion being a portion located on a skin side, the non-skin-side portion being a portion located on a non-skin side.

14. An absorbent article according to any one of claims 1 to 4, wherein
the groove includes
a main groove that is elongated in the predetermined direction that is the longitudinal direction, and
a sub groove that extends from the main groove in the width direction,
a plurality of the main grooves are arranged side-by-side spacing in the width direction, and
a plurality of the sub grooves extend from each of the main grooves in the width direction with located spacing in the longitudinal direction.

15. An absorbent article according to any one of claims 1 to 5, wherein
the upper-layer core includes a super absorbent polymer, and
the upper-layer core has a second groove in which a portion of the upper-layer core is recessed in the thickness direction,
the second groove is elongated in the longitudinal direction,
when viewed in the thickness direction, the upper-layer core has a second groove region in which the second groove is provided and
a second non-groove region in which the second groove is not provided,
when viewed in the thickness direction, at least a portion of the second groove region is provided in a region where the upper-layer core and the lower-layer core are overlapped with each other, and
when the upper-layer core is divided into two portions in the thickness direction,
an average density of the super absorbent polymer in a skin-side portion of the second groove region is lower than an average density of the super absorbent polymer in a skin-side portion of the second non-groove region,
the skin-side portion being a portion located on a skin side,
the non-skin-side portion being a portion located on a non-skin side.

16. An absorbent article according to claim 6, wherein
an average density of the super absorbent polymer in the non-skin-side portion of the second non-groove region is lower than an average density of the super absorbent polymer in the skin-side portion of the second non-groove region.

17. An absorbent article according to claim 6 or 7, wherein
the second groove is a groove that is recessed from a non-skin-side surface of the lower-layer core toward the skin side in the thickness direction.

18. An absorbent article according to any one of claims 6 to 8, wherein
the groove region of the lower-layer core has a portion that is overlapped with the second groove region of the upper-layer core when viewed in the thickness direction.

19. An absorbent article according to any one of claims 6 to 9, wherein
the lower-layer core has a slit in a widthwise central portion of a region of the lower-layer core that abuts a crotch portion of a wearer,
the slit penetrating the lower-layer core in the thickness direction,
the slit is elongated in the longitudinal direction, and
when viewed in the thickness direction,
the slit of the lower-layer core is overlapped with the upper-layer core, and
in at least a partial region of the slit of the lower-layer core, the slit is not overlapped with the second groove region of the upper-layer core.

20. An absorbent article according to claim 10, wherein
an elastic member is arranged in a region that abuts the crotch portion of the wearer,
the elastic member stretching and contracting in the longitudinal direction,
an entirety of the slit of the lower-layer core is not overlapped with the second groove region of the upper-layer core when viewed in the thickness direction, and
the slit has a portion overlapped with the elastic member when viewed in the thickness direction.

21. An absorbent article according to any one of claims 1 to 11, wherein
the lower-layer core has a pair of side slits in widthwise side portions of a region of the lower-layer core that abuts a crotch portion of a wearer,
the pair of side slits penetrating the lower-layer core in the thickness direction,
the groove region is provided at a position
that are outward in the width direction with respect to the pair of side slits,
that are at least partially overlapped with the pair of side slits in the longitudinal direction.

22. An absorbent article according to any one of claims 1 to 12, wherein
the lower-layer core has a slit in a widthwise central portion of a region of the lower-layer core that abuts a crotch portion of a wearer,
the slit penetrating the lower-layer core in the thickness direction,
the slit is elongated in the longitudinal direction,
the groove region is elongated in the predetermined direction that is the longitudinal direction,
at a position backward in the longitudinal direction with respect to the slit,
the lower-layer core has a region in which the non-groove region is continuous from one end to another end in the width direction, and
a back end of the upper-layer core in the longitudinal direction is located in the region in which the non-groove region is continuous.

23. An absorbent article according to any one of claims 1 to 13, wherein
the lower-layer core extends outward in the longitudinal direction from a longitudinal end of the upper-layer core, and
the groove region is provided in a portion of the lower-layer core that extends outward in the longitudinal direction from the upper-layer core.
